# EUROPEAN PATENT APPLICATION

(11) **EP 4 825 353 A2**
(43) Date of publication of application: **21.10.2026**
(21) Application number: 26194232.0
(22) Date of filing: 18.09.2020
(51) Int. Cl.: A61F 13/537

(54) **ABSORBENT NONWOVEN MATERIALS**

(30) Priority: 18.09.2019 US 201962902038 P; 18.09.2019 US 201962902051 P
(62) Document number(s) of the earlier application(s) in accordance with Art. 76 EPC: 24196249.7 / 4 442 882; 20781085.4 / 4 031 365
(71) Applicant: Glatfelter Corporation, Charlotte, NC 28209 (US)
(72) Inventor: DUTKIEWICZ, Jacek K., Cordova, 38016 (US); CAVANAUGH, Thomas J., Appleton, 54915 (US); FONG, Brian, Lakeland, 38002 (US)
(74) Representative: Grünecker Patent- und Rechtsanwälte PartG mbB

(57) **Abstract**

The presently disclosed subject matter relates to multi-layer nonwoven materials and their use in absorbent articles, e.g., as absorbent cores and/or acquisition distribution layers (ADL). More particularly, the presently disclosed subject matter relates to layered structures that provide for improved liquid acquisition, distribution, storage and rewet properties while allowing for the use of less synthetic materials, such as superabsorbent polymers (SAP) than other commercially available materials.

## Description

### CROSS-REFERENCE TO RELATED APPLICATIONS

This application claims the benefit of priority to U.S. Provisional Appln. Serial Nos. 62/902,038 and 62/902,051, both filed on September 18, 2019, the contents of each of which are hereby incorporated by reference in their entireties.

### 1. FIELD

The presently disclosed subject matter relates to new nonwoven materials and their use, e.g., as absorbent cores and/or acquisition distribution layers, in absorbent articles including personal hygiene products such as incontinence, light incontinence, and feminine hygiene products. More particularly, such structures provide for improved liquid acquisition, distribution, storage and rewet properties while allowing for the use of less synthetic materials, such as superabsorbent polymers (SAP), as compared to conventional absorbent cores or absorbent systems including separate acquisition distribution materials.

### 2. BACKGROUND

Nonwoven structures are important in a wide range of consumer products, such as absorbent articles including baby diapers, adult incontinence products, light incontinence products, feminine hygiene products such as panty liners, sanitary napkins, and the like. Such absorbent articles can require the fast acquisition of liquid. In certain nonwoven articles, there is often an absorbent core to receive and retain body liquids. The absorbent core is usually disposed between a liquid pervious topsheet, whose function is to allow the passage of fluid to the core and a liquid impervious backsheet whose function is to contain the fluid and to prevent it from passing through the absorbent article to the garment of the wearer of the absorbent article. In certain nonwoven articles, an acquisition-distribution layer (ADL) can be used in combination with the absorbent core. The ADL can facilitate both the acquisition of liquids and distribution of such liquids for storage in the absorbent core.

In the conventional multi-layer absorbent structures having an acquisition layer, a distribution layer and a storage layer, the acquisition layer acquires the liquid insult and quickly transmits it by capillary action away from the skin of the wearer (in the Z-direction). Next, the fluid encounters the distribution layer. The distribution layer is typically of a higher density material, and causes the liquid to migrate away from the skin of the wearer (in the Z-direction) and also laterally across the structure (in the X-Y directions). Finally, the liquid migrates into the storage layer. The storage layer generally includes high density cellulose fibers and superabsorbent polymer (SAP) particles. The liquid is absorbed by the storage layer and especially the SAP particles contained therein. In other conventional multi-layer absorbent structures having an acquisition layer and a storage layer, the acquisition layer acquires the liquid insult and distributes the liquid away from the skin of the wearer. The liquid migrates and is absorbed into the storage layer.

In recent years, there has been market and consumer demand for absorbent articles which require lesser amounts of synthetic materials, such as SAP, and are more economical. There has also been market and consumer demand for thinner absorbent articles. Additionally, there has been a need for increased performance of such nonwoven articles in terms of liquid acquisition, distribution, storage and rewet characteristics while also providing suitable dryness.

Thus, there remains a need for nonwoven materials that have enough absorbent capacity for its intended use and yet be conformable with the desired dryness profile. There also remains a need for economical and thinner absorbent articles containing lesser amounts of synthetic materials. The disclosed subject matter addresses these and other needs.

### 3. SUMMARY OF THE INVENTION

The presently disclosed subject matter provides for absorbent structures with multi-layer nonwoven materials containing specific layered constructions, which advantageously achieve increased liquid acquisition, distribution storage and rewet properties while allowing for the use of less synthetic materials, such as superabsorbent polymers (SAP), and reduced overall basis weight. In certain aspects, nonwoven materials of the present disclosure include a multi-layered absorbent core comprising a layer of bonded fine cellulosic fibers such as hardwood fibers and a layer of bonded coarse cellulosic fibers such as softwood fibers to impart improved characteristics with respect to liquid acquisition, distribution, and rewet of the nonwoven material and advantageously allow for the reduced incorporation of synthetic materials. In certain aspects, nonwoven materials of the present disclosure include an acquisition-distribution layer (ADL) having absorbent features of fibrous networks of bonded long fibers with those of bonded short fibers which advantageously provide for increased liquid storage and distribution performance.

Multi-layer nonwoven materials including at least two layers and absorbent articles including the same are provided. The nonwoven material can include a first layer and a second layer. The first layer can include cellulose fibers and bicomponent fibers. The second layer can be adjacent to the first layer and can include finer cellulose fibers and bicomponent fibers. At least a portion of the first and second layers can be coated with a binder.

In certain embodiments, the nonwoven material can have an effective acquisition time (EAT) of about 40 seconds or less or about 20 seconds or less. In certain embodiments, the nonwoven material can have a wicking distance of at least about 85 mm or at least about 140 mm. In certain embodiments, the nonwoven material can have a rewet value of about 0.2 g or less or about 0.15 g or less. In certain embodiments, the nonwoven material can have a retention before leak of at least about 3.0 g.

In certain embodiments, the fine cellulose fibers can include eucalyptus pulp.

In certain embodiments, the nonwoven material can further include an intermediate layer disposed between the first and second layers. The first intermediate layer can include superabsorbent polymer (SAP).

In certain embodiments, the nonwoven material can further include a second intermediate layer disclosed between the first layer and the first intermediate layer. The second intermediate layer can include cellulose fibers and bicomponent fibers.

Multi-layer nonwoven materials including at least four layers and absorbent articles including the same are provided. The nonwoven material can include a first layer, a second layer, a third layer, and a fourth layer. The first layer can include cellulose fibers and synthetic fibers. The second layer can be adjacent to the first layer and include cellulose fibers and synthetic fibers. The third layer can be adjacent to the second layer and can include superabsorbent polymer (SAP). The fourth layer can be adjacent to the third layer and can include fine cellulose fibers and synthetic fibers. At least a portion of the first and fourth layer can be coated with a binder.

In certain embodiments, the fine cellulose fibers can include eucalyptus pulp. In certain embodiments, the synthetic can comprise bicomponent fibers.

Multi-layer nonwoven materials having at least two layers and absorbent articles including the same are provided. The nonwoven material can include a first layer and a second layer. The first layer can include long fibers. The second layer can be adjacent to the first layer and include short fibers. At least a portion of the second layer can be coated with a binder.

In certain embodiments, the nonwoven material can have an effective acquisition time (EAT) of about 15 seconds or less or about 1 second or less. In certain embodiments, the nonwoven material can have a rewet value of about 0.5 g or less or about 0.05 g or less. In certain embodiments, the nonwoven material can have a wicking distance of at least about 140 mm or at least about 180 mm.

In certain embodiments, the long fibers can include synthetic fibers, regenerated cellulose fibers, or combinations thereof. In particular embodiments, the long fibers can include synthetic fibers formed as a carded web. The long fibers can have a length of between about 8 mm and about 70 mm. In certain embodiments, the short fibers can include synthetic fibers, cellulose fibers, regenerated cellulose fibers, or combinations thereof. The short fibers can have a length of between about 1 mm and about 8 mm.

Multi-layer nonwoven materials having at least two layers including a first layer including synthetic fibers formed as a carded web and absorbent articles including the same are provided. The nonwoven material can further include a second layer adjacent to the first layer. The second layer can include cellulose fibers. At least a portion of the second layer can be coated with a binder. In certain embodiments, the synthetic fibers can have a length of between about 8 mm and about 70 mm.

Multi-layer nonwoven materials including at least three layers and absorbent articles including the same are provided. The nonwoven material can include a first layer, a second layer, and a third layer. The first layer can include synthetic fibers formed as a carded web. The second layer can be adjacent to the first layer and can include cellulose fibers and bicomponent fibers. The third layer can be adjacent to the second layer and can include cellulose fibers and bicomponent fibers. At least a portion of the third layer can be coated with a binder. In certain embodiments, the synthetic fibers can have a length of between about 8 mm and about 70 mm. In certain embodiments, the cellulose fibers of the third layer can include fine cellulose fibers. In particular embodiments, the cellulose fibers of the third layer can include eucalyptus pulp.

Multi-layer nonwoven materials including at least four layers and absorbent articles including the same are provided. The nonwoven material can include a first layer, a second layer, a third layer, and a fourth layer. The first layer can include synthetic fibers formed as a carded web. The second layer can be adjacent to the first layer and can include cellulose fibers and bicomponent fibers. The third layer can be adjacent to the second layer and can include superabsorbent polymer (SAP). The fourth layer can be adjacent to the third layer and can include cellulose fibers and bicomponent fibers. At least a portion of the fourth layer can be coated with a binder. In certain embodiments, the synthetic fibers can have a length of between about 8 mm and about 70 mm. In certain embodiments, the cellulose fibers of the fourth layer can include fine cellulose fibers. In particular embodiments, the cellulose fibers of the fourth layer can include eucalyptus pulp.

The foregoing has outlined broadly the features and technical advantages of the present application in order that the detailed description that follows can be better understood.

Additional features and advantages of the application will be described hereinafter which form the subject of the claims of the application. It should be appreciated by those skilled in the art that the conception and specific embodiment disclosed can be readily utilized as a basis for modifying or designing other structures for carrying out the same purposes of the present application. It should also be realized by those skilled in the art that such equivalent constructions do not depart from the spirit and scope of the application as set forth in the appended claims. The novel features which are believed to be characteristic of the application, both as to its organization and method of operation, together with further objects and advantages will be better understood from the following description.

### 4. BRIEF DESCRIPTION OF THE DRAWINGS

FIG. 1 depicts the effective acquisition time test results of absorbent structures (Structures B, C, and D) prepared in accordance with certain non-limiting embodiments as compared to commercial absorbent structures, in accordance with Example 2;
FIG. 2 the rewet test results of absorbent structures (Structures B, C, and D) prepared in accordance with certain non-limiting embodiments as compared to commercial absorbent structures, in accordance with Example 2;
FIG. 3 depicts the effective acquisition time test results of absorbent structures (Structures C, D, F, H, I, J and K) prepared in accordance with certain non-limiting embodiments as compared to commercial absorbent structures, in accordance with Example 2;
FIG. 4 depicts the rewet test results of absorbent structures (Structures C, D, F, H, I, J and K) prepared in accordance with certain non-limiting embodiments as compared to commercial absorbent structures, in accordance with Example 2;
FIG. 5 depicts an apparatus used during fluid retention testing for retention before leak of absorbent structures, in accordance with Example 2;
FIGS. 6A-C depict the liquid retention test results as retention before leak of absorbent structures (Structures B, C, F, H, I, J and K) prepared in accordance with certain non-limiting embodiments as compared to commercial absorbent structures, in accordance with Example 2. FIG. 6A depicts the liquid retention test results of Structure B. FIG. 6B depicts the liquid retention test results of Structures C, H and I. FIG. 6C depicts the liquid retention test results of Structures F, J and K;
FIGS. 7A-C depict the wicking test results of absorbent structures (Structures B, C, F, H, I, J and K) prepared in accordance with certain non-limiting embodiments as compared to commercial absorbent structures, in accordance with Example 2. FIG. 7A depicts the wicking test results of Structure B. FIG. 7B depicts the wicking test results of Structures C, H and I. FIG. 7C depicts the wicking test results of Structures F, J and K;
FIG. 8 depicts the test effective acquisition time test results of absorbent structures prepared in accordance with certain non-limiting embodiments as compared to commercial absorbent structures, in accordance with Example 4;
FIG. 9 depicts the rewet test results of an absorbent structure (Structure 1A) prepared in accordance with certain non-limiting embodiments in a feminine sanitary napkin application as compared to commercial absorbent structures, in accordance with Example 4;
FIG. 10 depicts the rewet test results of an absorbent structure (Structure 1B) prepared in a diaper application accordance with certain non-limiting embodiments as compared to a commercial absorbent structure, in accordance with Example 5;
FIG. 11 depicts the basis weight of absorbent structures (Structures 2-4) prepared in accordance with certain non-limiting embodiments as compared to commercial absorbent structures, in accordance with Example 6;
FIG. 12 depicts the thickness of absorbent structures (Structures 2-4) prepared in accordance with certain non-limiting embodiments before and after 4 bar compression, in accordance with Example 6;
FIG. 13 depicts the effective acquisition time test results of absorbent structures (Structures 2-4) prepared in accordance with certain non-limiting embodiments as compared to commercial absorbent structures, in accordance with Example 6;
FIG. 14 depicts the rewet test results of absorbent structures (Structures 2-4) prepared in accordance with certain non-limiting embodiments as compared to commercial absorbent structures, in accordance with Example 6; and
FIG. 15 the wicking test results of absorbent structures (Structures 2-4) prepared in accordance with certain non-limiting embodiments as compared to commercial absorbent structures, in accordance with Example 6.

### 5. DETAILED DESCRIPTION

The presently disclosed subject matter provides multi-layer nonwoven materials for use, e.g., as absorbent cores and/or acquisition distribution layers (ADL), in absorbent articles. The presently disclosed subject matter also provides methods for making such materials. These and other aspects of the disclosed subject matter are discussed more in the detailed description and Examples.

### Definitions

The terms used in this specification generally have their ordinary meanings in the art, within the context of this subject matter and in the specific context where each term is used. Certain terms are defined below to provide additional guidance in describing the compositions and methods of the disclosed subject matter and how to make and use them.

As used in the specification and the appended claims, the singular forms "a," "an" and "the" include plural referents unless the context clearly dictates otherwise. Thus, for example, reference to "a compound" includes mixtures of compounds.

The term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to systems or processes, the term can mean within an order of magnitude, preferably within 5-fold, and more preferably within 2-fold, of a value.

The term "basis weight" as used herein refers to the quantity by weight of a compound over a given area. Examples of the units of measure include grams per square meter as identified by the acronym "gsm".

As used herein, the term "capillary action" refers to the ability of a liquid to flow in narrow spaces without the assistance of, or even in opposition to, external forces such as gravity. Section 2.1.3 "Surface Properties and Capillary Tension" of the Dutkiewicz, J., Nonwoven Structures for Absorption of Body Fluids, (2003) ISBN 2-930159-46-4 (published by Edana - Brussels, Belgium) publication provides additional disclosure with reference to capillary action.

As used herein, the term "cellulose" or "cellulosic" includes any material having cellulose as a major constituent, and specifically, comprising at least 50 percent by weight cellulose or a cellulose derivative. Thus, the term includes cotton, typical wood pulps, cellulose acetate, rayon, thermochemical wood pulp, chemical wood pulp, debonded chemical wood pulp, milkweed floss, microcrystalline cellulose, microfibrillated cellulose, and the like.

As used herein, the phrase "chemically modified," when used in reference to a fiber, means that the fiber has been treated with a polyvalent metal-containing compound to produce a fiber with a polyvalent metal-containing compound bound to it. It is not necessary that the compound chemically bond with the fibers, although it is preferred that the compound remain associated in close proximity with the fibers, by coating, adhering, precipitation, or any other mechanism such that it is not dislodged from the fibers during normal handling of the fibers. In particular, the compound can remain associated with the fibers even when wetted or washed with a liquid. For convenience, the association between the fiber and the compound can be referred to as the bond, and the compound can be said to be bound to the fiber.

As used herein, the term "fiber" or "fibrous" refers to a particulate material wherein the length to diameter ratio of such particulate material is greater than about 10. Conversely, a "nonfiber" or "nonfibrous" material is meant to refer to a particulate material wherein the length to diameter ratio of such particulate matter is about 10 or less.

As used herein, the term "hybrid" such as in "hybrid absorbent structures" or "hybrid absorbent materials" refers to a structure including a carded nonwoven web (e.g., TABCW) and an absorbent core.

As used herein, the term "liquid" refers to a substance having a fluid consistency. For example, and not limitation, liquids can include water, oils, solvents, bodily fluids such as urine or blood.

As used herein, the terms "long fibers" or "longer fibers" refers to fibers having a length of from about 8 mm to about 70 mm, including all intervening values. In certain embodiments, the long fibers can comprise synthetic fibers formed as a carded nonwoven web.

As used herein, a "nonwoven" refers to a class of material, including but not limited to textiles or plastics. Nonwovens are sheet or web structures made of fiber, filaments, molten plastic, or plastic films bonded together mechanically, thermally, or chemically. A nonwoven is a fabric made directly from a web of fiber, without the yarn preparation necessary for weaving or knitting. In a nonwoven, the assembly of fibers is held together by one or more of the following: (1) by mechanical interlocking in a random web or mat; (2) by fusing of the fibers, as in the case of thermoplastic fibers; or (3) by bonding with a cementing medium such as a natural or synthetic resin.

As used herein, the terms "short fibers" or "shorter fibers" refers to fibers having a length of from about 1 mm to about 8 mm, including all intervening values.

As used herein, the term "weight percent" is meant to refer to either (i) the quantity by weight of a constituent/component in the material as a percentage of the weight of a layer of the material; or (ii) to the quantity by weight of a constituent/component in the material as a percentage of the weight of the final nonwoven material or product.

### Fibers

The nonwoven material of the presently disclosed subject matter comprises fibers. In certain embodiments, the fibers can include long fibers, short fibers, or a mixture thereof. The fibers can be natural, synthetic, or a mixture thereof. In certain embodiments, the fibers can be cellulose-based fibers, one or more synthetic fibers, or a mixture thereof.

### Cellulose Fibers

Any cellulose fibers known in the art, including cellulose fibers of any natural origin, such as those derived from wood pulp or regenerated cellulose, can be used in a cellulosic layer. In certain embodiments, cellulose fibers include, but are not limited to, digested fibers, such as kraft, prehydrolyzed kraft, soda, sulfite, chemi-thermal mechanical, and thermomechanical treated fibers, derived from softwood, hardwood or cotton linters. Regenerated cellulose can be prepared by dissolving cellulose into monomers and regenerating a continuous cellulose polymer. The resultant polymer can be 100% cellulose, and can also be made into longer fibers, for example, for use in textiles, etc. In other embodiments, cellulose fibers include, but are not limited to, kraft digested fibers, including prehydrolyzed kraft digested fibers. Non-limiting examples of cellulose fibers suitable for use in this subject matter are the cellulose fibers derived from softwoods, such as pines, firs, and spruces. Other suitable cellulose fibers include, but are not limited to, those derived from Esparto grass, bagasse, kemp, flax, hemp, kenaf, and other lignaceous and cellulosic fiber sources. Suitable cellulose fibers include, but are not limited to, bleached Kraft southern pine fibers sold under the trademark FOLEY FLUFFS^{®} (Buckeye Technologies Inc., Memphis, Tenn.). Additionally, fibers sold under the trademark CELLU TISSUE^{®} (e.g., Grade 3024) (Clearwater Paper Corporation, Spokane, Wash.) are utilized in certain aspects of the disclosed subject matter.

The nonwoven materials of the disclosed subject matter can also include, but are not limited to, a commercially available bright fluff pulp including, but not limited to, southern softwood kraft (such as Golden Isles^{®} 4725 from GP Cellulose) or southern softwood fluff pulp (such as Treated FOLEY FLUFFS^{®}) northern softwood sulfite pulp (such as T 730 from Weyerhaeuser), or hardwood pulp (such as Eucalyptus). In certain embodiments, the nonwoven materials can include Eucalyptus fibers (Suzano, untreated). While certain pulps can be preferred based on a variety of factors, any absorbent fluff pulp or mixtures thereof can be used. In certain embodiments, wood cellulose, cotton linter pulp, chemically modified cellulose such as crosslinked cellulose fibers and highly purified cellulose fibers can be used. Non-limiting examples of additional pulps are FOLEY FLUFFS^{®} FFTAS (also known as FFTAS or Buckeye Technologies FFT-AS pulp), and Weyco CF401.

In certain embodiments, fine fibers, such as certain softwood fibers can be used. Certain non-limiting examples of such fine fibers, with pulp fiber coarseness properties are provided in Table I below with reference to Watson, P., et al., Canadian Pulp Fibre Morphology: Superiority and Considerations for End Use Potential, The Forestry Chronicle, Vol. 85 No. 3, 401-408 May/June 2009.

**Table I. Softwood Fibers**

| Species | Pulp Fiber Coarseness (mg/100 m) |
|---|---|
| Coastal Douglas-fir | 24 |
| Western hemlock | 20 |
| Spruce/pine | 18 |
| Western redcedar | 16 |
| Southern pine | 30 |
| Radiata pine | 22 |
| Scandinavian pine | 20 |
| Black spruce | 18 |

In certain embodiments, fine fibers, such as certain hardwood fibers can be used. Certain non-limiting examples of such fine fibers, with pulp fiber coarseness properties are provided in Table II with reference, at least in part, to Horn, R., Morphology of Pulp Fiber from Hardwoods and Influence on Paper Strength, Research Paper FPL 312, Forest Products Laboratory, U.S. Department of Agriculture (1978) and Bleached Eucalyptus Kraft Pulp ECF Technical Sheet (April 2017) (available at: https://www.metsafibre.com/en/Documents/Data-sheets/Cenibra-euca-Eucalyptus.pdf). In particular embodiments, Eucalyptus pulp can be used.

**Table II. Hardwood Fibers**

| Species | Pulp Fiber Coarseness (mg/100 m) |
|---|---|
| Red alder | 12.38 |
| Aspen | 8.59 |
| American elm | 9.53 |
| Paper birch | 13.08 |
| American beech | 13.10 |
| Shagbark hickory | 10.59 |
| Sugar maple | 7.86 |
| White oak | 14.08 |
| Eucalyptus | 6.5 +/- 2.3 |

Other suitable types of cellulose fiber include, but are not limited to, chemically modified cellulose fibers. In particular embodiments, the modified cellulose fibers are crosslinked cellulose fibers. U.S. Patent Nos. 5,492,759, 5,601,921, and 6,159,335, all of which are hereby incorporated by reference in their entireties, relate to chemically treated cellulose fibers useful in the practice of this disclosed subject matter. In certain embodiments, the modified cellulose fibers comprise a polyhydroxy compound. Non-limiting examples of polyhydroxy compounds include glycerol, trimethylolpropane, pentaerythritol, polyvinyl alcohol, partially hydrolyzed polyvinyl acetate, and fully hydrolyzed polyvinyl acetate. In certain embodiments, the fiber is treated with a polyvalent cation-containing compound. In one embodiment, the polyvalent cation-containing compound is present in an amount from about 0.1 weight percent to about 20 weight percent based on the dry weight of the untreated fiber. In particular embodiments, the polyvalent cation containing compound is a polyvalent metal ion salt. In certain embodiments, the polyvalent cation containing compound is selected from the group consisting of aluminum, iron, tin, salts thereof, and mixtures thereof. Any polyvalent metal salt including transition metal salts can be used. Non-limiting examples of suitable polyvalent metals include beryllium, magnesium, calcium, strontium, barium, titanium, zirconium, vanadium, chromium, molybdenum, tungsten, manganese, iron, cobalt, nickel, copper, zinc, aluminum and tin. Preferred ions include aluminum, iron and tin. The preferred metal ions have oxidation states of +3 or +4. Any salt containing the polyvalent metal ion can be employed. Non-limiting examples of suitable inorganic salts of the above metals include chlorides, nitrates, sulfates, borates, bromides, iodides, fluorides, nitrides, perchlorates, phosphates, hydroxides, sulfides, carbonates, bicarbonates, oxides, alkoxides phenoxides, phosphites, and hypophosphites. Non-limiting examples of suitable organic salts of the above metals include formates, acetates, butyrates, hexanoates, adipates, citrates, lactates, oxalates, propionates, salicylates, glycinates, tartrates, glycolates, sulfonates, phosphonates, glutamates, octanoates, benzoates, gluconates, maleates, succinates, and 4,5-dihydroxy-benzene-1,3-disulfonates. In addition to the polyvalent metal salts, other compounds such as complexes of the above salts include, but are not limited to, amines, ethylenediaminetetra-acetic acid (EDTA), diethylenetriaminepenta-acetic acid (DIPA), nitrilotri-acetic acid (NTA), 2,4-pentanedione, and ammonia can be used.

In one embodiment, the cellulose pulp fibers are chemically modified cellulose pulp fibers that have been softened or plasticized to be inherently more compressible than unmodified pulp fibers. The same pressure applied to a plasticized pulp web will result in higher density than when applied to an unmodified pulp web. Additionally, the densified web of plasticized cellulose fibers is inherently softer than a similar density web of unmodified fiber of the same wood type. Softwood pulps can be made more compressible using cationic surfactants as debonders to disrupt interfiber associations. Use of one or more debonders facilitates the disintegration of the pulp sheet into fluff in the airlaid process. Examples of debonders include, but are not limited to, those disclosed in U.S. Patent Nos. 4,432,833, 4,425,186 and 5,776,308, all of which are hereby incorporated by reference in their entireties. One example of a debonder-treated cellulose pulp is FFLE+. Plasticizers for cellulose, which can be added to a pulp slurry prior to forming wetlaid sheets, can also be used to soften pulp, although they act by a different mechanism than debonding agents. Plasticizing agents act within the fiber, at the cellulose molecule, to make flexible or soften amorphous regions. The resulting fibers are characterized as limp. Since the plasticized fibers lack stiffness, the comminuted pulp is easier to densify compared to fibers not treated with plasticizers. Plasticizers include, but are not limited to, polyhydric alcohols such as glycerol, low molecular weight polyglycol such as polyethylene glycols, and polyhydroxy compounds. These and other plasticizers are described and exemplified in U.S. Pat. Nos. 4,098,996, 5,547,541 and 4,731,269, all of which are hereby incorporated by reference in their entireties. Ammonia, urea, and alkylamines are also known to plasticize wood products, which mainly contain cellulose (A. J. Stamm, Forest Products Journal 5(6):413, 1955, hereby incorporated by reference in its entirety).

In particular embodiments of the disclosed subject matter, the following cellulose is used: GP4723, a fully treated pulp (Leaf River) (available from Georgia-Pacific); GP4725, a semi-treated pulp (available from Georgia-Pacific); Tencel (available from Lenzing); cellulose flax fibers; Danufil (available from Kelheim); Viloft (available from Kelheim); GP4865, an odor control semi-treated pulp (available from Georgia-Pacific); Grade 3024 Cellu Tissue (available from Clearwater); Brawny Industrial Flax 500 (available from Georgia-Pacific).

Nonwoven materials of the present disclosure can include cellulose fibers. In certain embodiments, one or more layers of the nonwoven material can contain from about 5 gsm to about 150 gsm, about 5 gsm to about 100 gsm, or about 10 gsm to about 50 gsm cellulose fibers. In particular embodiments, one or more layers can contain about 18 gsm, about 25 gsm, about 54 gsm, about 62 gsm, about 69 gsm, or about 70 gsm cellulose fibers.

### Synthetic Fibers

In addition to the use of cellulose fibers, the presently disclosed subject matter also contemplates the use of synthetic fibers. In one embodiment, the synthetic fibers comprise bicomponent and/or mono-component fibers. Bicomponent fibers having a core and sheath are known in the art. Many varieties are used in the manufacture of nonwoven materials, particularly those produced for use in airlaid techniques. Various bicomponent fibers suitable for use in the presently disclosed subject matter are disclosed in U.S. Patent Nos. 5,372,885 and 5,456,982, both of which are hereby incorporated by reference in their entireties. Examples of bicomponent fiber manufacturers include, but are not limited to, Trevira (Bobingen, Germany), Fiber Innovation Technologies (Johnson City, TN) and ES Fiber Visions (Athens, GA).

Bicomponent fibers can incorporate a variety of polymers as their core and sheath components. Bicomponent fibers that have a PE (polyethylene) or modified PE sheath typically have a PET (polyethylene terephthalate) or PP (polypropylene) core. In one embodiment, the bicomponent fiber has a core made of polyester and sheath made of polyethylene. In another embodiment, the bicomponent fiber has a core made of polypropylene and a sheath made of polyethylene.

The denier of the bicomponent fiber preferably ranges from about 1.0 dpf to about 4.0 dpf, and more preferably from about 1.5 dpf to about 2.5 dpf. The length of the bicomponent fiber can be from about 3 mm to about 36 mm, preferably from about 3 mm to about 12 mm, more preferably from about 3 mm to about 10. In particular embodiments, the length of the bicomponent fiber is from about 4 mm to about 8 mm, or about 6 mm. In a particular embodiment, the bicomponent fiber is Trevira T255 which contains a polyester core and a polyethylene sheath modified with maleic anhydride. T255 has been produced in a variety of deniers, cut lengths and core sheath configurations with preferred configurations having a denier from about 1.7 dpf to 2.0 dpf and a cut length of about 4 mm to 12 mm and a concentric core sheath configuration. In a specific embodiment, the bicomponent fiber is Trevira 1661, T255, 2.0 dpf and 6 mm in length. In an alternate embodiment, the bicomponent fiber is Trevira 1663, T255, 2.0 dpf and 3 mm in length.

Bicomponent fibers are typically fabricated commercially by melt spinning. In this procedure, each molten polymer is extruded through a die, for example, a spinneret, with subsequent pulling of the molten polymer to move it away from the face of the spinneret. This is followed by solidification of the polymer by heat transfer to a surrounding fluid medium, for example chilled air, and taking up of the now solid filament. Non-limiting examples of additional steps after melt spinning can also include hot or cold drawing, heat treating, crimping and cutting. This overall manufacturing process is generally carried out as a discontinuous two-step process that first involves spinning of the filaments and their collection into a tow that comprises numerous filaments. During the spinning step, when molten polymer is pulled away from the face of the spinneret, some drawing of the filament does occur which can also be called the draw-down. This is followed by a second step where the spun fibers are drawn or stretched to increase molecular alignment and crystallinity and to give enhanced strength and other physical properties to the individual filaments. Subsequent steps can include, but are not limited to, heat setting, crimping and cutting of the filament into fibers. The drawing or stretching step can involve drawing the core of the bicomponent fiber, the sheath of the bicomponent fiber or both the core and the sheath of the bicomponent fiber depending on the materials from which the core and sheath are comprised as well as the conditions employed during the drawing or stretching process.

Bicomponent fibers can also be formed in a continuous process where the spinning and drawing are done in a continuous process. During the fiber manufacturing process it is desirable to add various materials to the fiber after the melt spinning step at various subsequent steps in the process. These materials can be referred to as "finish" and be comprised of active agents such as, but not limited to, lubricants and anti-static agents. The finish is typically delivered via an aqueous based solution or emulsion. Finishes can provide desirable properties for both the manufacturing of the bicomponent fiber and for the user of the fiber, for example in an airlaid or wetlaid process.

Numerous other processes are involved before, during and after the spinning and drawing steps and are disclosed in U.S. Patent Nos. 4,950,541, 5,082,899, 5,126,199, 5,372,885, 5,456,982, 5,705,565, 2,861,319, 2,931,091, 2,989,798, 3,038,235, 3,081,490, 3,117,362, 3,121,254, 3,188,689, 3,237,245, 3,249,669, 3,457,342, 3,466,703, 3,469,279, 3,500,498, 3,585,685, 3,163,170, 3,692,423, 3,716,317, 3,778,208, 3,787,162, 3,814,561, 3,963,406, 3,992,499, 4,052,146, 4,251,200, 4,350,006, 4,370,114, 4,406,850, 4,445,833, 4,717,325, 4,743,189, 5,162,074, 5,256,050, 5,505,889, 5,582,913, and 6,670,035, all of which are hereby incorporated by reference in their entireties.

The presently disclosed subject matter can also include, but are not limited to, articles that contain bicomponent fibers that are partially drawn with varying degrees of draw or stretch, highly drawn bicomponent fibers and mixtures thereof. These can include, but are not limited to, a highly drawn polyester core bicomponent fiber with a variety of sheath materials, specifically including a polyethylene sheath such as Trevira T255 (Bobingen, Germany) or a highly drawn polypropylene core bicomponent fiber with a variety of sheath materials, specifically including a polyethylene sheath such as ES FiberVisions AL-Adhesion-C (Varde, Denmark). Additionally, Trevira T265 bicomponent fiber (Bobingen, Germany), having a partially drawn core with a core made of polybutylene terephthalate (PBT) and a sheath made of polyethylene can be used. The use of both partially drawn and highly drawn bicomponent fibers in the same structure can be leveraged to meet specific physical and performance properties based on how they are incorporated into the structure.

The bicomponent fibers of the presently disclosed subject matter are not limited in scope to any specific polymers for either the core or the sheath as any partially drawn core bicomponent fiber can provide enhanced performance regarding elongation and strength. The degree to which the partially drawn bicomponent fibers are drawn is not limited in scope as different degrees of drawing will yield different enhancements in performance. The scope of the partially drawn bicomponent fibers encompasses fibers with various core sheath configurations including, but not limited to concentric, eccentric, side by side, islands in a sea, pie segments and other variations. The relative weight percentages of the core and sheath components of the total fiber can be varied. In addition, the scope of this subject matter covers the use of partially drawn homopolymers such as polyester, polypropylene, nylon, and other melt spinnable polymers. The scope of this subject matter also covers multicomponent fibers that can have more than two polymers as part of the fiber structure.

Nonwoven materials of the present disclosure can include bicomponent fibers. In certain embodiments, one or more layers of the nonwoven material can contain from about 1 gsm to about 40 gsm, about 5 gsm to about 30 gsm, or about 10 gsm to about 25 gsm bicomponent fibers. In particular embodiments, one or more layers of the nonwoven material can contain about 8 gsm, about 12 gsm, about 21 gsm, about 25 gsm, or about 27 gsm of bicomponent fibers. In alternative embodiments, the bicomponent layer contains from about 10 gsm to about 50 gsm, about 12 gsm to about 40 gsm, or about 20 gsm to about 30 gsm bicomponent fibers.

In particular embodiments, the bicomponent fibers are low dtex staple bicomponent fibers in the range of about 0.5 dtex to about 20 dtex. In certain embodiments, the dtex value can range from about 1.3 dtex to about 15 dtex, or from about 1.5 dtex to about 10 dtex, or from about 1.7 dtex to about 6.7 dtex, or from about 2.2 dtex to about 5.7 dtex. In certain embodiments, the dtex value is 1.3 dtex, 1.5 dtex, 1.7 dtex, 2.2 dtex, 3.3 dtex, 5.7 dtex, 6.7 dtex, or 10 dtex.

Other synthetic fibers suitable for use in various embodiments as fibers or as bicomponent binder fibers include, but are not limited to, fibers made from various polymers including, by way of example and not by limitation, acrylic, polyamides (including, but not limited to, Nylon 6, Nylon 6/6, Nylon 12, polyaspartic acid, polyglutamic acid), polyamines, polyimides, polyacrylics (including, but not limited to, polyacrylamide, polyacrylonitrile, esters of methacrylic acid and acrylic acid), polycarbonates (including, but not limited to, polybisphenol A carbonate, polypropylene carbonate), polydienes (including, but not limited to, polybutadiene, polyisoprene, polynorbomene), polyepoxides, polyesters (including, but not limited to, polyethylene terephthalate, polybutylene terephthalate, polytrimethylene terephthalate, polycaprolactone, polyglycolide, polylactide, polyhydroxybutyrate, polyhydroxyvalerate, polyethylene adipate, polybutylene adipate, polypropylene succinate), polyethers (including, but not limited to, polyethylene glycol (polyethylene oxide), polybutylene glycol, polypropylene oxide, polyoxymethylene (paraformaldehyde), polytetramethylene ether (polytetrahydrofuran), polyepichlorohydrin), polyfluorocarbons, formaldehyde polymers (including, but not limited to, urea-formaldehyde, melamine-formaldehyde, phenol formaldehyde), natural polymers (including, but not limited to, cellulosics, chitosans, lignins, waxes), polyolefins (including, but not limited to, polyethylene, polypropylene, polybutylene, polybutene, polyoctene), polyphenylenes (including, but not limited to, polyphenylene oxide, polyphenylene sulfide, polyphenylene ether sulfone), silicon containing polymers (including, but not limited to, polydimethyl siloxane, polycarbomethyl silane), polyurethanes, polyvinyls (including, but not limited to, polyvinyl butyral, polyvinyl alcohol, esters and ethers of polyvinyl alcohol, polyvinyl acetate, polystyrene, polymethylstyrene, polyvinyl chloride, polyvinyl pryrrolidone, polymethyl vinyl ether, polyethyl vinyl ether, polyvinyl methyl ketone), polyacetals, polyarylates, and copolymers (including, but not limited to, polyethylene-co-vinyl acetate, polyethylene-co-acrylic acid, polybutylene terephthalate-co-polyethylene terephthalate, polylauryllactam-block-polytetrahydrofuran), polybutylene succinate and polylactic acid based polymers.

In specific embodiments, the synthetic fiber layer contains a high dtex staple fibers in the range of about 2 to about 20 dtex. In certain embodiments, the dtex value can range from about 2 dtex to about 15 dtex, or from about 2 dtex to about 10 dtex. In particular embodiments, the fiber can have a dtex value of about 6.7 dtex.

In other specific embodiments, the synthetic layer contains synthetic filaments. The synthetic filaments can be formed by spinning and/or extrusion processes. For example, such processes can be similar to the methods described above with reference to melt spinning processes. The synthetic filaments can include one or more continuous strands. In certain embodiments, the synthetic filaments can include polypropylene.

### Short Fibers

In certain embodiments, nonwoven materials of the present disclosure can include at least one layer comprising short fibers. Short fibers can provide high capillary action to nonwoven materials and increased distribution of liquid. Short fibers suitable for use in the nonwoven materials of the present disclosure can include cellulose fibers, regenerated cellulose fibers, synthetic fibers, and combinations thereof as disclosed herein. Short fibers can include fibers having a length of from about 1 mm to about 8 mm, about 2 mm to about 8 mm, about 3 mm to about 6 mm, or about 5 mm to about 6 mm. In particular embodiments, the short fibers can include fibers having a length of about 1 mm, about 2 mm, about 3 mm, about 4 mm, about 5 mm, about 6 mm, about 7 mm, or about 8 mm.

Nonwoven materials of the present disclosure can include short fibers. In certain embodiments, one or more layers of the nonwoven material can contain from about 5 gsm to about 150 gsm, about 5 gsm to about 100 gsm, or about 10 gsm to about 50 gsm short fibers. In particular embodiments, one or more layers can contain about 15 gsm, about 30 gsm, about 75 gsm, or about 100 gsm short fibers.

### Long Fibers

In certain embodiments, nonwoven materials of the present disclosure can include at least one layer comprising long fibers. Long fibers can provide for increased liquid acquisition of liquid in a relatively short amount of time. Long fibers suitable for use in the nonwoven materials of the present disclosure can include regenerated cellulose fibers, synthetic fibers, and combinations thereof as disclosed herein. Long fibers can include fibers having a length of from about 8 mm to about 70 mm, about 10 mm to about 60 mm, about 15 mm to about 50 mm, or about 20 mm to about 40 mm. In particular embodiments, the long fibers can include fibers having a length of about 8 mm, about 10 mm, about 30 mm, about 50 mm, about 55 mm, about 60 mm, about 65 mm, or about 70 mm.

Nonwoven materials of the present disclosure can include long fibers. In certain embodiments, one or more layers of the nonwoven material can contain from about 10 gsm to about 150 gsm, about 10 gsm to about 100 gsm, about 20 gsm to about 60 gsm, about 25 gsm to about 50 gsm, or about 30 gsm to about 35 gsm of long fibers. In particular embodiments, nonwoven materials can include about 10 gsm, about 20 gsm, about 25 gsm, about 30 gsm, about 34 gsm, about 40 gsm, about 45 gsm, about 50 gsm, about 75 gsm, about 100 gsm, about 120 gsm, or about 150 gsm of long fibers.

### Carded Webs

In certain embodiments, nonwoven materials of the present disclosure can include a carded web comprising fibers. Such carded webs can include longer fibers which can be synthetic fibers. The term "longer fibers" as used herein refers to fibers whose length is between about 8 mm and about 70 mm. Examples of carded webs suitable for use with the present disclosure include Carded Nonwoven Web (TABCW) (Product Code STACT8H34) (Shalag Nonwovens, Oxford, NC). Carded webs including longer fibers are generally more resilient than conventional airlaid webs, however, nonwoven materials with longer fibers generally do not have acceptable liquid distribution and storage properties.

In certain embodiments, nonwoven materials can include at least one layer comprising synthetic fibers formed as a carded web in an amount of from about 10 gsm to about 150 gsm, about 10 gsm to about 100 gsm, about 20 gsm to about 60 gsm, about 25 gsm to about 50 gsm, or about 30 gsm to about 35 gsm. In particular embodiments, nonwoven materials can include at least one layer comprising synthetic fibers as carded webs in an amount of about 10 gsm, about 20 gsm, about 25 gsm, about 30 gsm, about 34 gsm, about 40 gsm, about 45 gsm, about 50 gsm, about 75 gsm, about 100 gsm, about 120 gsm, or about 150 gsm.

### Binders

In certain embodiments, the nonwoven materials described herein can include binders. Suitable binders include, but are not limited to, liquid binders and powder binders. Non-limiting examples of liquid binders include emulsions, solutions, or suspensions of binders. Non-limiting examples of binders include polyethylene powders, copolymer binders, vinylacetate ethylene binders, styrene-butadiene binders, urethanes, urethane-based binders, acrylic binders, thermoplastic binders, natural polymer based binders, and mixtures thereof.

Suitable binders include, but are not limited to, copolymers, vinylacetate ethylene ("VAE") copolymers, which can have a stabilizer such as Wacker Vinnapas 192, Wacker Vinnapas EF 539, Wacker Vinnapas EP907, Wacker Vinnapas EP129, Celanese Duroset E130, Celanese Dur-O-Set Elite 130 25-1813 and Celanese Dur-O-Set TX-849, Celanese 75-524A, polyvinyl alcohol-polyvinyl acetate blends such as Wacker Vinac 911, vinyl acetate homopolyers, polyvinyl amines such as BASF Luredur, acrylics, cationic acrylamides, polyacryliamides such as Bercon Berstrength 5040 and Bercon Berstrength 5150, hydroxyethyl cellulose, starch such as National Starch CATO RTM 232, National Starch CATO RTM 255, National Starch Optibond, National Starch Optipro, or National Starch OptiPLUS, guar gum, styrene-butadienes, urethanes, urethane-based binders, thermoplastic binders, acrylic binders, and carboxymethyl cellulose such as Hercules Aqualon CMC. In certain embodiments, the binder is a natural polymer based binder. Non-limiting examples of natural polymer based binders include polymers derived from starch, cellulose, chitin, and other polysaccharides.

In certain embodiments, the binder is water-soluble. In one embodiment, the binder is a vinylacetate ethylene copolymer. One non-limiting example of such copolymers is EP907 (Wacker Chemicals, Munich, Germany). Vinnapas EP907 can be applied at a level of about 10% solids incorporating about 0.75% by weight Aerosol OT (Cytec Industries, West Paterson, N.J.), which is an anionic surfactant. Other classes of liquid binders such as styrene-butadiene and acrylic binders can also be used. In certain embodiments, Vinnapas 192 can be applied at a level of about 15% incorporating about 0.08% by weight Aerosol OT 75 (Cytec Industries, West Paterson, N.J.).

In certain embodiments, the binder is not water-soluble. Examples of these binders include, but are not limited to, Vinnapas 124 and 192 (Wacker), which can have an opacifier and whitener, including, but not limited to, titanium dioxide, dispersed in the emulsion. Other binders include, but are not limited to, Celanese Emulsions (Bridgewater, N.J.) Elite 22 and Elite 33.

In certain embodiments, the binder is a thermoplastic binder. Such thermoplastic binders include, but are not limited to, any thermoplastic polymer which can be melted at temperatures which will not extensively damage the cellulose fibers. Preferably, the melting point of the thermoplastic binding material will be less than about 175 °C. Examples of suitable thermoplastic materials include, but are not limited to, suspensions of thermoplastic binders and thermoplastic powders. In particular embodiments, the thermoplastic binding material can be, for example, polyethylene, polypropylene, polyvinylchloride, and/or polyvinylidene chloride.

In particular embodiments, the vinylacetate ethylene binder is non-crosslinkable. In one embodiment, the vinylacetate ethylene binder is crosslinkable. In certain embodiments, the binder is WD4047 urethane-based binder solution supplied by HB Fuller. In one embodiment, the binder is Michem Prime 4983-45N dispersion of ethylene acrylic acid ("EAA") copolymer supplied by Michelman. In certain embodiments, the binder is Dur-O-Set Elite 22LV emulsion of VAE binder supplied by Celanese Emulsions (Bridgewater, N.J.). As noted above, in particular embodiments, the binder is crosslinkable. It is also understood that crosslinkable binders are also known as permanent wet strength binders. A permanent wet-strength binder includes, but is not limited to, Kymene^{®} (Hercules Inc., Wilmington, Del.), Parez^{®} (American Cyanamid Company, Wayne, N.J.), Wacker Vinnapas or AF192 (Wacker Chemie AG, Munich, Germany), or the like. Various permanent wet-strength agents are described in U.S. Patent Nos. 2,345,543, 2,926,116, and 2,926,154, the disclosures of which are incorporated by reference in their entirety. Other permanent wet-strength binders include, but are not limited to, polyamine-epichlorohydrin, polyamide epichlorohydrin or polyamideamine epichlorohydrin resins, which are collectively termed "PAE resins". Non-limiting exemplary permanent wet-strength binders include Kymene 557H or Kymene 557LX (Hercules Inc., Wilmington, DE) and have been described in U.S. Patent Nos. 3,700,623 and 3,772,076, which are incorporated herein in their entirety by reference thereto.

Alternatively, in certain embodiments, the binder is a temporary wet-strength binder. The temporary wet-strength binders include, but are not limited to, Hercobond^{®} (Hercules Inc., Wilmington, Del.), Parez^{®} 750 (American Cyanamid Company, Wayne, N.J.), Parez^{®} 745 (American Cyanamid Company, Wayne, N.J.), or the like. Other suitable temporary wet-strength binders include, but are not limited to, dialdehyde starch, polyethylene imine, mannogalactan gum, glyoxal, and dialdehyde mannogalactan. Other suitable temporary wet-strength agents are described in U.S. Patent Nos. 3,556,932, 5,466,337, 3,556,933, 4,605,702, 4,603,176, 5,935,383, and 6,017,417, all of which are incorporated herein in their entirety by reference thereto.

In certain embodiments, binders can be applied as emulsions in amounts ranging from about 1 gsm to about 10 gsm, about 1 gsm to about 8 gsm, about 1 gsm to about 5 gsm, about 1 gsm to about 4 gsm, about 5 gsm to about 10 gsm, about 2 gsm to about 5 gsm, or about 2 gsm to about 3 gsm. In particular embodiments, binders can be applied as emulsions in an amount of about 1 gsm, about 2 gsm, about 3 gsm, about 4 gsm, or about 5 gsm. Binders can be applied to one side of a fibrous layer, preferably an externally facing layer. Alternatively, binder can be applied to both sides of a layer, in equal or disproportionate amounts. In certain embodiments, binders can be applied to at least one outer surface of a nonwoven material.

### Other Additives

The materials of the presently disclosed subject matter can also contain other additives. For example, the materials can contain superabsorbent polymer (SAP). The types of superabsorbent polymers which can be used in the disclosed subject matter include, but are not limited to, SAPs in their particulate form such as powder, irregular granules, spherical particles, staple fibers and other elongated particles. In certain embodiments, the materials can contain superabsorbent fibers (SAF; manufactured by Technical Absorbents Limited, 9 dtex, 5.8 mm). U.S. Patent Nos. 5,147,343, 5,378,528, 5,795,439, 5,807,916, 5,849,211, and 6,403,857, which are hereby incorporated by reference in their entireties, describe various superabsorbent polymers and methods of making superabsorbent polymers. One example of a superabsorbent polymer forming system is crosslinked acrylic copolymers of metal salts of acrylic acid and acrylamide or other monomers such as 2-acrylamido-2-methylpropanesulfonic acid. Many conventional granular superabsorbent polymers are based on poly(acrylic acid) which has been crosslinked during polymerization with any of a number of multi-functional co-monomer crosslinking agents well-known in the art. Examples of multi-functional crosslinking agents are set forth in U.S. Patent Nos. 2,929,154, 3,224,986, 3,332,909, and 4,076,673, which are incorporated herein by reference in their entireties. For instance, crosslinked carboxylated polyelectrolytes can be used to form superabsorbent polymers. Other water-soluble polyelectrolyte polymers are known to be useful for the preparation of superabsorbents by crosslinking, these polymers include: carboxymethyl starch, carboxymethyl cellulose, chitosan salts, gelatine salts, etc. They are not, however, commonly used on a commercial scale to enhance absorbency of dispensable absorbent articles mainly due to their higher cost. Superabsorbent polymer granules useful in the practice of this subject matter are commercially available from a number of manufacturers, such as BASF, Dow Chemical (Midland, Mich.), Stockhausen (Greensboro, N.C.), Chemdal (Arlington Heights, Ill.), and Evonik (Essen, Germany). Non-limiting examples of SAP include a surface crosslinked acrylic acid based powder such as Stockhausen 9350 or SX70, BASF Hysorb Fem 33, BASF HySorb FEM 33N, or Evonik Favor SXM 7900.

In particular embodiments, the SAP can be starch-based. For example, the SAP can include K-Boost (XGF-450, manufactured by Corno Cascades LLC, Beavertown, OR) or K-Boost (XGF 463, manufactured by Corno Cascades LLC, Beavertown, OR). Such starched-based SAPs can be biodegradable. In certain embodiments, the SAP can include a high capacity SAP, a high-speed SAP, or combinations thereof. Particular examples of a high capacity SAP include K-Boost (XGF-450, manufactured by Corno Cascades LLC, Beavertown, OR). Particular examples of a high-speed SAP include K-Boost (XGF 463, manufactured by Corno Cascades LLC, Beavertown, OR).

In certain embodiments, SAP can be used in a layer in amounts ranging from about 5% to about 50% based on the total weight of the structure. In certain embodiments, the content of SAP is between about 0% and about 30%, about 0% and about 15%, about 5% and about 25%, about 5% and about 15%, or about 10% and about 20%, based on a total weight of the structure. In particular embodiments, the content of SAP is about 0%, about 2%, about 5%, about 8%, about 10%, about 15%, about 20%, about 25%, or about 30%, based on a total weight of the structure. In certain embodiments, the amount of SAP in a layer can range from about 5 gsm to about 50 gsm, about 5 gsm to about 25 gsm, about 10 gsm to about 50 gsm, or about 12 gsm to about 40 gsm, or about 15 gsm to about 25 gsm. In particular embodiments, SAP can be used in a layer in an amount of about 10 gsm or about 20 gsm.

### Nonwoven Materials

The presently disclosed subject matter provides for improved nonwoven materials with several advantages over various commercially available materials. The presently disclosed materials have a achieved improved overall absorbency performance in terms of liquid acquisition, distribution and rewet as compared to conventional materials with higher content of synthetic materials, such as superabsorbent polymer (SAP). Nonwoven materials of the present disclosure therefore advantageously provide increased absorbency performance with lower amounts of synthetic additives. In certain aspects, the presently disclosed materials have a significantly reduced absorbent mass, with an ability to achieve comparable or improved overall absorbency performance. The absorbency performance is measured by increased fluid acquisition or improved dryness characteristics, while maintaining low basis weights to commercially available products. Further, nonwoven materials of the present disclosure provide for the use of lesser amounts of synthetic materials, such as SAP, as compared to various commercially available materials.

Nonwoven materials of the present disclosure can be used with various components in absorbent systems. In certain aspects, nonwoven materials of the present disclosure can be used as an absorbent core in absorbent systems, for example, in personal care products such as light incontinence products and feminine hygiene products, such as panty liners. Such absorbent cores can have a specific multi-layer structure comprising a layer of bonded, fine cellulosic fibers such as hardwood fibers, for example, Eucalyptus fibers. The structure can further include a layer of bonded coarse cellulosic fibers such as softwood fibers. Optionally, the structure can further include superabsorbent polymer (SAP). Such nonwoven materials, when combined with a conventional acquisition distribution layer (ADL) such as a carded layer, can exhibit increased performance in terms of liquid acquisition, distribution, and rewet compared to conventional absorbent cores with higher SAP content. In certain aspects, nonwoven materials of the present disclosure can be used as acquisition distribution layers (ADL) in absorbent systems, for example, in personal care products such as feminine hygiene products and light incontinence products. As conventional airlaid nonwovens with bonded short fibers (i.e., between about 1 mm and about 8 mm) generally lack high enough liquid acquisition speed characteristics and do not provide enough dryness relative to other nonwovens, such as carded materials, which use longer (i.e., between about 8 mm and about 70 mm), synthetic fibers. As provided above, carded webs having longer fibers are generally more resilient than conventional airlaid webs, however, nonwovens with longer fibers generally do not have acceptable liquid distribution and storage properties. In certain aspects, nonwoven materials of the present disclosure provide for absorbent features of fibrous networks of bonded long fibers with those of bonded short fibers or networks of bonded short fibers with embedded superabsorbent polymer in the form of powders (SAP) or fibers (SAF). Such nonwoven materials surprisingly and advantageously provide for increase liquid storage and distribution performance. In certain aspects, nonwoven materials of the present disclosure provide a unitary multi-layer hybrid liquid acquisition distribution (ADL) material with increased fluid intake and rewet performance than conventional airlaids. Such structures combine bonded carded webs with one or more layers of airlaid webs in specific configurations and fiber contents. Other aspects of the present disclosure provide for unitary multifunctional hybrid absorbent structures with improved liquid acquisition, distribution, storage and rewet characteristics. In addition to a layer of bonded longer fibers providing for faster fluid intake and increased rewet performance, structures of the present disclosure can also include a liquid storage layer and a distribution layer. The storage layer can optionally include SAP and/or SAF and the distribution layer can include bonded fine cellulosic fibers such as hardwood fibers, for example, Eucalyptus fibers. Such nonwoven materials surprisingly and advantageously provide increased liquid acquisition, distribution, storage and rewet characteristics while allowing for a reduced basis weight of the absorbent system and use of less synthetic materials such as SAP relative to conventional multicomponent absorbent systems comprising a separate conventional acquisition distribution layer (ADL) materials and liquid storage absorbent components.

The presently disclosed subject matter provides for nonwoven materials. In certain embodiments, the nonwoven material includes at least two layers, at least three layers, or at least four layers.

In certain embodiments, the nonwoven material can include at least two layers, wherein each layer comprises a specific fibrous content. In specific embodiments, the nonwoven material can be a two-layer nonwoven structure. The nonwoven material can include a first layer comprising cellulose fibers and synthetic fibers. The nonwoven material can further include a second layer adjacent to the first layer. The second layer can include cellulose fibers and synthetic fibers. The first and second layers can be bonded on at least a portion of their outer surface with a binder. In certain embodiments, the synthetic fibers can include bicomponent fibers. In certain embodiments, the cellulose fibers of the second layer can include fine cellulose fibers. In particular embodiments, the cellulose fibers of the second layer can include Eucalyptus pulp.

In certain embodiments, the nonwoven material can include a first layer comprising long fibers. The nonwoven material can further include a second layer adjacent to the first layer. The second layer can include short fibers. The second layer can be bonded on at least a portion of its outer surface with a binder. In certain embodiments, the long fibers can include synthetic fibers. In particular embodiments, the long fibers can include synthetic fibers formed as a carded web. In certain embodiments, the short fibers can include cellulose fibers.

In certain embodiments, the nonwoven material can include at least three layers, wherein each layer comprises a specific fibrous content. In specific embodiments, the nonwoven material can be a three-layer nonwoven structure. The nonwoven material can include a first layer including cellulose fibers and synthetic fibers. The nonwoven material can further include a second layer adjacent to the first layer. The second layer can include superabsorbent polymer (SAP). The nonwoven material can further include a third layer adjacent to the second layer. The third layer can include cellulose fibers and synthetic fibers. The first and third layers can be bonded on at least a portion of their outer surface with a binder. In certain embodiments, the synthetic fibers can include bicomponent fibers. In certain embodiments, the cellulose fibers of the third layer can include fine cellulose fibers. In particular embodiments, the cellulose fibers of the third layer can include Eucalyptus pulp.

In certain embodiments, the nonwoven material can include a first layer comprising long fibers. The nonwoven material can further include a second layer adjacent to the first layer. The second layer can include short fibers. The nonwoven material can further include a third layer adjacent to the second layer. The third layer can include short fibers. The third layer can be bonded on at least a portion of its outer surface with a binder. In certain embodiments, the long fibers can include synthetic fibers. In particular embodiments, the long fibers can include synthetic fibers formed as a carded web. In certain embodiments, the short fibers of the second and third layers can include a blend of short fibers. For example, and not by way of limitation, the short fibers of the second and third layers can include synthetic fibers and cellulose fibers. In certain embodiments, the synthetic fibers of the second and third layers can include bicomponent fibers. In certain embodiments, the cellulose fibers of the third layer can include Eucalyptus pulp.

In certain embodiments, the nonwoven material can include at least four layers, wherein each layer comprises a specific fibrous content. In specific embodiments, the nonwoven material can be a four-layer nonwoven structure. The nonwoven material can include a first layer including cellulose fibers and synthetic fibers. The nonwoven material can further include a second layer adjacent to the first layer. The second layer can include cellulose fibers and synthetic fibers. The nonwoven material can further include a third layer adjacent to the second layer. The third layer can include superabsorbent polymer (SAP). The nonwoven material can further include a fourth layer adjacent to the third layer. The fourth layer can include cellulose fibers and synthetic fibers. The first and fourth layers can be bonded on at least a portion of their outer surface with a binder. In certain embodiments, the synthetic fibers can include bicomponent fibers. In certain embodiments, the cellulose fibers of the fourth layer can include fine cellulose fibers. In particular embodiments, the cellulose fibers of the fourth layer can include Eucalyptus pulp.

In certain embodiments, the nonwoven material can include a first layer comprising long fibers. The nonwoven material can further include a second layer adjacent to the first layer. The second layer can include short fibers. The nonwoven material can further include a third layer adjacent to the second layer. The third layer can include additional additives such as super absorbent polymer (SAP). The nonwoven material can further include a fourth layer adjacent to the third layer. The fourth layer can include short fibers. The fourth layer can be bonded on at least a portion of its outer surface with a binder. In certain embodiments, the long fibers of the first layer can include synthetic fibers. In particular embodiments, the long fibers of the first layer can include synthetic fibers formed as a carded web. In certain embodiments, the short fibers of the second and fourth layers can include a blend of short fibers. For example, and not by way of limitation, the short fibers of the second and fourth layers can include synthetic fibers and cellulose fibers. In certain embodiments, the synthetic fibers of the second and fourth layers can include bicomponent fibers. In certain embodiments, the cellulose fibers of the fourth layer can include Eucalyptus pulp.

Nonwoven materials of the present disclosure can include at least two layers, at least three layers, or at least four layers, wherein each layer comprises a specific fibrous content. In certain embodiments, the first layer can include a blend of cellulose fibers and synthetic fibers. In certain embodiments, the synthetic fibers of the first layer can include bicomponent fibers. The cellulosic fibers can be present in the first layer in an amount of about 20 gsm to about 70 gsm, about 30 gsm to about 60 gsm, or about 40 gsm to about 50 gsm. In particular embodiments, the first layer can include about 25 gsm, about 30 gsm, about 40 gsm, about 54 gsm, about 62 gsm, or about 70 gsm cellulose fibers. The synthetic fibers can be present in the first layer in an amount of about 15 gsm to about 40 gsm, about 20 gsm to about 35 gsm, or about 25 gsm to about 35 gsm. In particular embodiments, the first layer can include about 12 gsm, about 21 gsm, about 25 gsm, or about 30 gsm synthetic fibers.

In certain embodiments, the first layer can include long fibers in an amount of about 20 gsm to about 60 gsm, about 25 gsm to about 50 gsm, or about 30 gsm to about 35 gsm. In particular embodiments, the first layer can include long fibers in an amount of about 20 gsm, about 25 gsm, about 30 gsm, about 34 gsm, about 40 gsm, about 45 gsm, or about 50 gsm. In certain embodiments, the first layer can include synthetic fibers formed as a carded web in an amount of about 20 gsm to about 60 gsm, about 25 gsm to about 50 gsm, or about 30 gsm to about 35 gsm. In particular embodiments, the first layer can include synthetic fibers formed as a carded web in an amount of about 20 gsm, about 25 gsm, about 30 gsm, about 34 gsm, about 40 gsm, about 45 gsm, or about 50 gsm.

In certain embodiments, the nonwoven materials can include a second layer. The second layer can include a blend of cellulose fibers and synthetic fibers. In certain embodiments, the synthetic fibers of the second layer can include bicomponent fibers. In particular embodiments, the cellulose fibers of the second layer can comprise fine cellulose fibers, such as Eucalyptus pulp. The cellulose fibers can be present in the second layer in an amount of about 5 gsm to about 70 gsm, about 10 gsm to about 65 gsm, or about 15 gsm to about 50 gsm. In particular embodiments, the second layer can include about 20 gsm, about 25 gsm, about 50 gsm, about 62 gsm, or about 65 gsm of cellulose fibers. The synthetic fibers can be present in the second layer in an amount of about 15 gsm to about 40 gsm, about 20 gsm to about 35 gsm, or about 25 gsm to about 35 gsm. In particular embodiments, the second layer can include about 8 gsm, about 12 gsm, about 20 gsm, or about 25 gsm synthetic fibers. In alternative embodiments, the second layer can include superabsorbent polymer (SAP). For example, and not by way of limitation, the second layer can include about 5 gsm to about 30 gsm, about 15 gsm to about 25 gsm, or about 10 gsm to about 20 gsm of superabsorbent polymer (SAP). In certain embodiments, the second layer can include about 10 gsm or about 20 gsm of superabsorbent polymer (SAP).

In certain embodiments, the second layer can include short fibers. In particular embodiments, the second layer can include cellulose fibers. In alternative embodiments, the second layer can include a blend of cellulose fibers and synthetic fibers such as bicomponent fibers. In certain embodiments, the second layer can include from about 5 gsm to about 100 gsm, about 10 gsm to about 80 gsm, or about 15 gsm to about 75 gsm of short fibers. In particular embodiments, the second layer can include about 16 gsm, about 18 gsm, about 75 gsm, about 85 gsm, or about 95 gsm of short fibers. The cellulose fibers can be present in the second layer in an amount of about 5 gsm to about 70 gsm, about 10 gsm to about 65 gsm, or about 15 gsm to about 50 gsm. In particular embodiments, the second layer can include about 18 gsm, about 25 gsm, about 50 gsm, about 54 gsm, about 62 gsm, or about 69 gsm of cellulose fibers. The synthetic fibers can be present in the second layer in an amount of about 15 gsm to about 40 gsm, about 20 gsm to about 35 gsm, or about 25 gsm to about 35 gsm. In particular embodiments, the second layer can include about 8 gsm, about 12 gsm, about 21 gsm, about 23 gsm, about 25 gsm, or about 26 gsm synthetic fibers.

In certain embodiments, the nonwoven materials can include a third layer. The third layer can include superabsorbent polymer (SAP). Alternatively, in certain embodiments, the third layer can include a blend of cellulose fibers and synthetic fibers. In particular embodiments, the cellulose fibers of the third layer can include fine cellulose fibers, such as Eucalyptus pulp. In certain embodiments, the synthetic fibers of the third layer can include bicomponent fibers. The cellulose fibers can be present in the third layer in an amount of about 5 gsm to about 70 gsm, about 10 gsm to about 65 gsm, or about 15 gsm to about 50 gsm. In particular embodiments, the third layer can include about 25 gsm, about 35 gsm, about 50 gsm, or about 62 gsm cellulose fibers. The synthetic fibers can be present in the third layer in an amount of about 15 gsm to about 40 gsm, about 20 gsm to about 35 gsm, or about 25 gsm to about 35 gsm. In particular embodiments, the third layer can include about 8 gsm, about 12 gsm, about 21 gsm, about 23 gsm, about 25 gsm, or about 26 gsm synthetic fibers. In alternative embodiments, the third layer can include superabsorbent polymer (SAP). For example, and not by way of limitation, the third layer can include about 5 gsm to about 30 gsm, about 15 gsm to about 25 gsm, or about 10 gsm to about 20 gsm of superabsorbent polymer (SAP). In certain embodiments, the third layer can include about 10 gsm or about 20 gsm of superabsorbent polymer (SAP).

In certain embodiments, the nonwoven materials can include a third layer. The third layer can include short fibers. In certain embodiments, the third layer can include a blend of cellulose fibers and synthetic fibers such as bicomponent fibers. In particular embodiments, the cellulose fibers of the third layer can include fine cellulose fibers, such as Eucalyptus pulp. In certain embodiments, the third layer can include from about 5 gsm to about 100 gsm, about 10 gsm to about 80 gsm, or about 15 gsm to about 75 gsm of short fibers. In particular embodiments, the third layer can include about 50 gsm, about 55 gsm, about 70 gsm, or about 75 gsm of short fibers. The cellulose fibers can be present in the third layer in an amount of about 5 gsm to about 70 gsm, about 10 gsm to about 65 gsm, or about 15 gsm to about 50 gsm. In particular embodiments, the third layer can include about 25 gsm, about 35 gsm, about 50 gsm, or about 62 gsm cellulose fibers. The synthetic fibers can be present in the third layer in an amount of about 15 gsm to about 40 gsm, about 20 gsm to about 35 gsm, or about 25 gsm to about 35 gsm. In particular embodiments, the third layer can include about 8 gsm, about 12 gsm, about 21 gsm, about 23 gsm, about 25 gsm, or about 26 gsm synthetic fibers. In particular embodiments, the third layer can include superabsorbent polymer (SAP). For example, and not by way of limitation, the third layer can include about 5 gsm to about 30 gsm, about 15 gsm to about 25 gsm, or about 10 gsm to about 20 gsm of superabsorbent polymer (SAP). In certain embodiments, the third layer can include about 10 gsm or about 20 gsm of superabsorbent polymer (SAP).

In certain embodiments, the nonwoven materials can include a fourth layer. The fourth layer can include a blend of cellulose fibers and synthetic fibers. In particular embodiments, the cellulose fibers of the fourth layer can include fine cellulose fibers, such as Eucalyptus pulp. In certain embodiments, the synthetic fibers of the fourth layer can include bicomponent fibers. The cellulose fibers can be present in the fourth layer in an amount of about 5 gsm to about 70 gsm, about 10 gsm to about 65 gsm, or about 15 gsm to about 50 gsm. In particular embodiments, the fourth layer can include about 25 gsm, about 35 gsm, about 50 gsm, or 62 gsm cellulose fibers. The synthetic fibers can be present in the fourth layer in an amount of about 15 gsm to about 40 gsm, about 20 gsm to about 35 gsm, or about 25 gsm to about 35 gsm. In particular embodiments, the fourth layer can include about 8 gsm, about 15 gsm, about 20 gsm, or about 25 gsm of synthetic fibers.

In certain embodiments, the nonwoven materials can include a fourth layer. The fourth layer can include short fibers. In certain embodiments, the fourth layer can include a blend of cellulose fibers and synthetic fibers such as bicomponent fibers. In particular embodiments, the cellulose fibers of the fourth layer can include fine cellulose fibers, such as Eucalyptus pulp. In certain embodiments, the fourth layer can include from about 5 gsm to about 100 gsm, about 10 gsm to about 80 gsm, or about 15 gsm to about 75 gsm of short fibers. In particular embodiments, the fourth layer can include about 50 gsm, about 55 gsm, about 70 gsm, or about 75 gsm of short fibers. The cellulose fibers can be present in the fourth layer in an amount of about 5 gsm to about 70 gsm, about 10 gsm to about 65 gsm, or about 15 gsm to about 50 gsm. In particular embodiments, the third layer can include about 25 gsm, about 35 gsm, about 50 gsm, or 62 gsm cellulose fibers. The synthetic fibers can be present in the fourth layer in an amount of about 15 gsm to about 40 gsm, about 20 gsm to about 35 gsm, or about 25 gsm to about 35 gsm. In particular embodiments, the fourth layer can include about 8 gsm, about 12 gsm, about 21 gsm, about 23 gsm, about 25 gsm, or about 26 gsm synthetic fibers.

In certain embodiments, at least one outer surface of the nonwoven material can be coated with a binder. Binders can be applied as emulsions in amounts ranging from about 1 gsm to about 10 gsm, about 1 gsm to about 8 gsm, about 1 gsm to about 5 gsm, about 1 gsm to about 4 gsm, about 5 gsm to about 10 gsm, about 2 gsm to about 5 gsm, or about 2 gsm to about 3 gsm. In particular embodiments, binders can be applied as emulsions in an amount of about 1 gsm, about 2 gsm, about 3 gsm, about 4 gsm, or about 5 gsm.

### Absorbent Cores and Acquisition Distribution Layers

In another aspect, the presently disclosed subject matter provides for a multi-layer nonwoven materials, e.g., absorbent cores, containing at least one layer adjacent to an acquisition distribution layer. In certain aspects, the presently disclosed subject matter provides for multi-layer nonwoven materials, e.g., acquisition distribution layers (ADL), containing at least one layer adjacent to an absorbent core. Such nonwoven materials of the present disclosure can be used with various other materials to provide absorbent systems. Hybrid systems of the present disclosure can include an acquisition distribution layer bonded to a core, thus, both the acquisition layer and a core can be provided in a single structured hybrid system.

Nonwoven materials of the present disclosure as absorbent cores in an absorbent system including an acquisition distribution layer such as a carded layer also provide increased performance in terms of liquid acquisition, distribution and rewet compared to conventional absorbent cores with higher SAP content. In certain embodiments, the nonwoven materials of the present disclosure can be used in an absorbent system include a topsheet. In certain embodiments, nonwoven materials of the present disclosure as absorbent cores can have a total basis weight of greater than about 100 gsm, between about 100 gsm to about 500 gsm, or between about 150 gsm to about 300 gsm.

Nonwoven materials of the present disclosure as an acquisition distribution layer in an absorbent system including an absorbent core provide hybrid absorbent structures with improved liquid acquisition, distribution and storage properties in one unitary structure. In certain embodiments, the absorbent system can have a total basis weight of about 200 gsm to about 250 gsm, about 210 gsm to about 225 gsm, or about 200 gsm to about 220 gsm. In particular embodiments, the absorbent system can have a total basis weight of about 214 gsm, about 218 gsm, or about 221 gsm.

### Features of the Nonwoven Materials

The presently disclosed nonwoven materials can have improved liquid acquisition properties. A person having ordinary skill in the art will appreciate that absorbency characteristics of a nonwoven material, can vary. For example, the observed absorbency characteristics can vary based on the amount of fluid and the surface area of the nonwoven material. Additionally, when the nonwoven materials contain an absorbent core and/or an acquisition distribution layer, the materials can have improved fluid acquisition characteristics. Furthermore, the nonwoven materials of the presently disclosed subject matter can quickly absorb a fluid. The use of bicomponent fibers in the nonwoven materials of the present disclosure can provide faster acquisition time. Further, the use of fine cellulose fibers, such as Eucalyptus pulp, in a bottom layer of the nonwoven materials can provide for improved liquid distribution. Such fine cellulose fibers can also provide for increased fluid retention and therefore lower rewet.

In certain embodiments, a nonwoven material as described above can absorb a fluid in less than about 60 seconds, less than about 45 seconds, less than about 40 seconds, less than about 30 seconds, less than about 15 seconds, less than about 8 seconds, or less than about 1 second. In particular embodiments, the nonwoven materials can absorb a fluid in about 0.8 seconds, about 1 second, about 1.2 seconds, about 1.4 seconds, about 2.7 seconds, about 3.2 seconds, about 4.9 seconds, about 7.1 seconds, about 8 seconds, or about 15 seconds. The time it takes for a material to absorb a fluid can be called an "acquisition time." For example, and not limitation, the acquisition time can be measured using the procedures described in Examples 2, 4 and 6 below. Furthermore, the presently disclosed nonwoven materials can acquire and retain more liquid before leakages occurs. In certain embodiments, the nonwoven materials can acquire and retain at least about 4.0 g, at least about 3.5 g, or at least 3.0 g prior to leakage of the liquid.

Furthermore, the presently disclosed nonwoven materials can have improved dryness characteristics, indicating improved fluid retention. For example, after absorbing a fluid, the nonwoven materials can be pressed to measure the amount of fluid released. In certain embodiments, a rewet test or a humidity sensation test can be used to press the nonwoven material and measure the released fluid, as described in various examples below. In certain embodiments, less than about 3 g, less than about 2 g, less than about 1.5 g, less than about 1 g, less than about 0.5 g, less than about 0.20 g, less than about 0.15 g, less than about 0.12 g, less than about 0.10 g, less than about 0.08 g, or less than about 0.06 g is released. In particular embodiments, less than about 2.8 g, less than about 0.11 g or less than about 0.05 g is released. Even with lower basis weights as compared to conventional products, nonwoven materials of the present disclosure contained no to relatively low amounts of SAP and provided increased rewet performance.

The presently disclosed nonwoven materials can have improved liquid distribution characteristics. In certain embodiments, nonwoven materials can provide a longer wicking distance as compared to conventional products which provide a dryer feel and increased comfort to the end user in terms of dryness. Longer wicking distances provide for increased utilization of absorbent materials, for example, in personal hygiene products during use. In certain embodiments, nonwoven materials of the present disclosure can have a wicking distance of from about 80 mm to about 200 mm, about 80 mm to about 160 mm, about 90 mm to about 150 mm, or about 100 to about 125 mm. In particular embodiments, nonwoven materials of the present disclosure can have a wicking distance of at least about 80 mm, at least about 85 mm, at least about 100 mm, at least about 125 mm, at least about 140 mm, at least about 148 mm, at least about 180 mm or at least about 190 mm.

In certain embodiments of the presently disclosed subject matter, at least a portion of at least one outer layer is coated with binder. In particular embodiments of the disclosed subject matter, at least a portion of each outer layer is coated with binder. In particular embodiments, the first and third layers are coated with a binder in amounts ranging from about 1 gsm to about 10 gsm, about 1 gsm to about 8 gsm, about 1 gsm to about 5 gsm, about 1 gsm to about 4 gsm, about 5 gsm to about 10 gsm, about 2 gsm to about 5 gsm, or about 2 gsm to about 3 gsm.

In certain embodiments of the nonwoven material, the range of basis weight of the overall structure is from about 5 gsm to about 600 gsm, or from about 5 gsm to about 400 gsm, or from about 10 gsm to about 400 gsm, or from about 20 gsm to 300 gsm, or from about 10 gsm to about 200 gsm, or from about 20 gsm to about 200 gsm, or from about 30 gsm to about 200 gsm, or from about 40 gsm to about 200 gsm. In certain embodiments where an absorbent core is present, the range of basis weight of the overall structure can be from about 10 gsm to about 1000 gsm, or from about 50 gsm to about 800 gsm, or from about 100 gsm to about 600 gsm.

The caliper of the nonwoven material refers to the caliper of the entire nonwoven material, inclusive of all layers. In certain embodiments, the caliper of the material ranges from about 0.5 mm to about 8.0 mm, or from about 0.5 mm to about 4 mm, or from about 0.5 mm to about 3.0 mm, or from about 0.5 mm to about 2.0 mm, or from about 0.7 mm to about 1.5 mm.

### Methods of Making the Nonwoven Materials

A variety of processes can be used to assemble the materials used in the practice of this disclosed subject matter to produce the materials, including but not limited to, traditional dry forming processes such as airlaying and carding or other forming technologies such as spunlace or airlace. Preferably, the materials can be prepared by airlaid processes. Airlaid processes include, but are not limited to, the use of one or more forming heads to deposit raw materials of differing compositions in selected order in the manufacturing process to produce a product with distinct strata. This allows great versatility in the variety of products which can be produced.

In one embodiment, the material is prepared as a continuous airlaid web. The airlaid web is typically prepared by disintegrating or defiberizing a cellulose pulp sheet or sheets, typically by hammermill, to provide individualized fibers. Rather than a pulp sheet of virgin fiber, the hammermills or other disintegrators can be fed with recycled airlaid edge trimmings and off-specification transitional material produced during grade changes and other airlaid production waste. Being able to thereby recycle production waste would contribute to improved economics for the overall process. The individualized fibers from whichever source, virgin or recycled, are then air conveyed to forming heads on the airlaid web-forming machine. A number of manufacturers make airlaid web forming machines suitable for use in the disclosed subject matter, including Dan-Web Forming of Aarhus, Denmark, M&J Fibretech A/S of Horsens, Denmark, Rando Machine Corporation, Macedon, N.Y. which is described in U.S. Patent No. 3,972,092, Margasa Textile Machinery of Cerdanyola del Valles, Spain, and DOA International of Wels, Austria. While these many forming machines differ in how the fiber is opened and air-conveyed to the forming wire, they all are capable of producing the webs of the presently disclosed subject matter. The Dan-Web forming heads include rotating or agitated perforated drums, which serve to maintain fiber separation until the fibers are pulled by vacuum onto a foraminous forming conveyor or forming wire. In the M&J machine, the forming head is basically a rotary agitator above a screen. The rotary agitator can comprise a series or cluster of rotating propellers or fan blades. Other fibers, such as a synthetic thermoplastic fiber, are opened, weighed, and mixed in a fiber dosing system such as a textile feeder supplied by Laroche S. A. of Cours-La Ville, France. In particular embodiments, such airlaid machines can be equipped with customized forming heads or heads capable of layer individualized longer fibers. From the textile feeder, the fibers are air conveyed to the forming heads of the airlaid machine where they are further mixed with the comminuted cellulose pulp fibers from the hammer mills and deposited on the continuously moving forming wire. Where defined layers are desired, separate forming heads can be used for each type of fiber. Alternatively or additionally, one or more layers can be prefabricated prior to being combined with additional layers, if any.

The airlaid web is transferred from the forming wire to a calendar or other densification stage to densify the web, if necessary, to increase its strength and control web thickness. In one embodiment, the fibers of the web are then bonded by passage through an oven set to a temperature high enough to fuse the included thermoplastic or other binder materials. In a further embodiment, secondary binding from the drying or curing of a latex spray or foam application occurs in the same oven. The oven can be a conventional through-air oven, be operated as a convection oven, or can achieve the necessary heating by infrared or even microwave irradiation. In particular embodiments, the airlaid web can be treated with additional additives before or after heat curing.

In certain aspects, nonwoven materials of the present disclosure including at least one layer comprising long fibers and at least one layer comprising short fibers can be prepared in accordance with various methods. In certain embodiments, the short fibers of at least one layer can be air-laid onto the at least one layer comprising long fibers. In particular embodiments, the long fibers can be formed in a carded web. Thus, the nonwoven material can have a structure including long fibers in one layer bonded to short fibers in another layer. In alternative embodiments, such nonwoven materials can be prepared by utilizing a forming head (e.g., manufactured by Campen Technology) to lay down long fibers onto a layer comprising short fibers.

### Applications and End Uses

The nonwoven materials of the disclosed subject matter can be used for any application known in the art. For example, the nonwoven materials can be used either alone or as a component, e.g., as an absorbent core and/or an acquisition distribution layer (ADL), in a variety of absorbent articles. In certain aspects, the nonwoven materials can be used in absorbent articles that absorb and retain body fluids. Such absorbent articles include baby diapers, adult incontinence products, light incontinence products and feminine hygiene products such as panty liners, sanitary napkins, and the like.

In other aspects, the nonwoven materials can be used alone or as a component, e.g., as an absorbent core and/or an acquisition distribution layer (ADL), in other consumer products. For example, the nonwoven materials can be used in absorbent cleaning products, such wipes, sheets, towels and the like. By way of example, the nonwoven materials can be used as a disposable wipe for cleaning applications, including household, personal, and industrial cleaning applications. The absorbency of the nonwoven materials can aid in dirt and mess removal in such cleaning applications. In particular aspects, the nonwoven materials can be used as filtration media.

### 6. EXAMPLES

The following examples are merely illustrative of the presently disclosed subject matter and they should not be considered as limiting the scope of the subject matter in any way.

### EXAMPLE 1: Absorbent Structures

The present Example provides absorbent structures that can provide improved acquisition, distribution and storage functions. The absorbent structures can be used as absorbent cores, for example, as a component of personal hygiene products.

Structure B was a nonwoven substrate including a multi-layer core structure to provide permanent storage with the use of superabsorbent polymer (SAP). Structure B was formed on a Danweb airlaid production line. The first layer included 25 gsm of cellulose fibers (Georgia-Pacific, GP 4723, fully treated pulp, Leaf River) blended with 12 gsm bicomponent fibers (Trevira, PE/PET 70% core, 1.7 dtex, 6mm). The next layer included 25 gsm of cellulose fibers (Georgia-Pacific, GP 4723, fully treated pulp, Leaf River) blended with 12 gsm bicomponent fibers (Trevira, PE/PET 70% core, 1.7 dtex, 6mm). The next layer included 20 gsm of SAP (BASF, HySorb Fem 33N) provided on top of the previous layer. The top layer included 62 gsm of Eucalyptus pulp (Suzano, untreated) mixed with 8 gsm of bicomponent fibers (Trevira, PE/PET 70% core, 1.7 dtex, 6mm). The Eucalyptus layer and outer fluff layer were each sprayed with 5 gsm (by dry weight) of a polymeric binder in the form of an emulsion (Vinnapas 192, Wacker with 0.8% Aerosol OT 75 surfactant). The total basis weight of Structure B was 174 gsm. Structure B included about 11.5% SAP.

The composition of Structure B is shown in Table 1.

**Table 1. Structure B Composition**

| Layer | Type of Material | Raw Materials | Basis Weight (gsm) |
|---|---|---|---|
| | Binder | Wacker Vinnapas 192 w/ 0.8% Aerosol OT 75 | 5.00 |
| 1 | Pulp | Fully-Treated GP 4723 | 25.00 |
| | Synthetic | T255 PE/PET 70% Core, 1.7 dtex, 6mm | 12.00 |
| 2 | Pulp | Fully-Treated GP 4723 | 25.00 |
| | Synthetic | T255 PE/PET 70% Core, 1.7 dtex, 6mm | 12.00 |
| 3 | SAP | BASF Hysorb Fem 33 | 20.00 |
| 4 | Pulp | Eucalyptus (Suzano, untreated) | 62.00 |
| | Synthetic | T255 PE/PET 70% Core, 1.7 dtex, 6mm | 8.00 |
| | Binder | Wacker Vinnapas 192 w/ 0.8% Aerosol OT 75 | 5.00 |
| | | Total | 174.00 |

Structure C was a nonwoven substrate including a multi-layer core structure to provide permanent storage with the use of superabsorbent polymer (SAP). Structure C was formed on a Danweb airlaid pilot line. The first layer included 62 gsm of Eucalyptus pulp (Suzano, untreated) mixed with 8 gsm bicomponent fibers (Trevira, PE/PET 30% core, 1.5 dtex, 6mm) and deposited on a forming wire. The next layer included 10 gsm of SAP (Evonik Favor SXM 7900) provided on top of the previous layer. The next layer included 62 gsm of cellulose fibers (Georgia-Pacific, GP 4723, fully treated pulp) blended with 25 gsm bicomponent fibers (Trevira, PE/PET 70% core, 1.7 dtex, 6mm). The top and bottom layers were sprayed with 4 gsm (by dry weight) of a polymeric binder in the form of an emulsion (Vinnapas 192, Wacker with 0.8% Aerosol OT 75 surfactant). The total basis weight of Structure C was 175 gsm. Structure C included about 5.7% SAP.

The composition of Structure C is shown in Table 2.

**Table 2. Structure C Composition**

| Layer | Type of Material | Raw Materials | Basis Weight (gsm) |
|---|---|---|---|
| | Binder | Wacker Vinnapas 192 w/ 0.8% Aerosol OT 75 | 4.00 |
| 1 | Pulp | Fully-Treated GP 4723 | 62.00 |
| | Synthetic | T255 PE/PET 70% Core, 1.7 dtex, 6mm | 25.00 |
| 2 | SAP | Evonik Favor SXM 7900 | 10.00 |
| 3 | Pulp | Eucalyptus (Suzano, untreated) | 62.00 |
| | Synthetic | T255 PE/PET 30% core, 1.5 dtex, 6mm | 8.00 |
| | Binder | Wacker Vinnapas 192 w/ 0.8% Aerosol OT 75 | 4.00 |
| | | Total | 175.00 |

Structure D was a nonwoven substrate including a multi-layer core structure to provide permanent storage. Structure D was formed on a Danweb airlaid pilot line. The first layer included 62 gsm of Eucalyptus pulp (Suzano, untreated) mixed with 8 gsm bicomponent fibers (Trevira, PE/PET 30% core, 1.5 dtex, 6mm) and deposited on a forming wire. The next layer included 70 gsm of cellulose fibers (Georgia-Pacific, GP 4723, fully treated pulp) blended with 27 gsm bicomponent fibers (Trevira, PE/PET 70% core, 1.7 dtex, 6mm). The top and bottom layers were sprayed with 4 gsm (by dry weight) of a polymeric binder in the form of an emulsion (Vinnapas 192, Wacker with 0.8% Aerosol OT 75 surfactant). The basis weight of Structure D was 175 gsm. Structure D did not include SAP.

The composition of Structure D is shown in Table 3.

**Table 3. Structure D Composition**

| Layer | Type of Material | Raw Materials | Basis Weight (gsm) |
|---|---|---|---|
| | Binder | Wacker Vinnapas 192 w/ 0.8% Aerosol OT 75 | 4.00 |
| 1 | Pulp | Fully-Treated GP 4723 | 70.00 |
| | Synthetic | T255 PE/PET 70% Core, 1.7 dtex, 6mm | 27.00 |
| 2 | Pulp | Eucalyptus (Suzano, untreated) | 62.00 |
| | Synthetic | T255 PE/PET 30% core, 1.5 dtex, 6mm | 8.00 |
| | Binder | Wacker Vinnapas 192 w/ 0.8% Aerosol OT 75 | 4.00 |
| | | Total | 175.00 |

Structure F was a nonwoven substrate including a multi-layer core structure to provide permanent storage with the use of superabsorbent polymer (SAP). Structure F was formed on a Danweb airlaid pilot line. The first layer included 62 gsm of Eucalyptus pulp (Suzano, untreated) mixed with 8 gsm bicomponent fibers (Trevira, PE/PET 30% core, 1.5 dtex, 6mm) and deposited on a forming wire. The next layer included 20 gsm SAP (Evonik Favor SXM 7900) provided on top of the previous layer. The next layer included 54 gsm of cellulose fibers (Georgia-Pacific, GP 4723, fully treated pulp) blended with 21 gsm bicomponent fibers (Trevira, PE/PET 70% core, 1.7 dtex, 6mm). The top and bottom layers were sprayed with 5 gsm (by dry weight) of a polymeric binder in the form of an emulsion (Vinnapas 192, Wacker with 0.8% Aerosol OT 75 surfactant). The basis weight of Structure F was 175 gsm. Structure F included about 11.5% SAP.

The composition of Structure F is shown in Table 4.

**Table 4. Structure F Composition**

| Layer | Type of Material | Raw Materials | Basis Weight (gsm) |
|---|---|---|---|
| | Binder | Wacker Vinnapas 192 w/ 0.8% Aerosol OT 75 | 5.00 |
| 1 | Pulp | Fully-Treated GP 4723 | 54.00 |
| | Synthetic | T255 PE/PET 70% Core, 1.7 dtex, 6mm | 21.00 |
| 2 | SAP | Evonik Favor SXM 7900 | 20.00 |
| 3 | Pulp | Eucalyptus (Suzano, untreated) | 62.00 |
| | Synthetic | T255 PE/PET 30% core, 1.5 dtex, 6mm | 8.00 |
| | Binder | Wacker Vinnapas 192 w/ 0.8% Aerosol OT 75 | 5.00 |
| | | Total | 175.00 |

Structure H was a nonwoven substrate including a multi-layer core structure to provide permanent storage with the use of superabsorbent polymer (SAP). Structure H was formed on a Danweb airlaid pilot line. The first layer included 62 gsm of Eucalyptus pulp (Suzano, untreated) mixed with 8 gsm bicomponent fibers (Trevira, PE/PET 30% core, 1.5 dtex, 6mm) and deposited on a forming wire. The next layer included 10 gsm of a starch-based SAP 1, K-Boost (XGF-450, manufactured by Como Cascades LLC, Beavertown, OR) provided on top of the previous layer. The next layer included 62 gsm of cellulose fibers (Georgia-Pacific, GP 4723, fully treated pulp) blended with 25 gsm bicomponent fibers (Trevira, PE/PET 70% core, 1.7 dtex, 6mm). The top and bottom layers were sprayed with a 4 gsm (by dry weight) of a polymeric binder in the form of an emulsion (Vinnapas 192, Wacker with 0.8% Aerosol OT 75 surfactant). The basis weight of Structure H was 175 gsm. Structure H included about 5.7% SAP.

The composition of Structure H is shown in Table 5.

**Table 5. Structure H Composition**

| Layer | Type of Material | Raw Materials | Basis Weight (gsm) |
|---|---|---|---|
| | Binder | Wacker Vinnapas 192 w/ 0.8% Aerosol OT 75 | 4.00 |
| 1 | Pulp | Fully-Treated GP 4723 | 62.00 |
| | Synthetic | T255 PE/PET 70% Core, 1.7 dtex, 6mm | 25.00 |
| 2 | SAP | Starch-based SAP 1, K-Boost (XGF-450) | 10.00 |
| 3 | Pulp | Eucalyptus (Suzano, untreated) | 62.00 |
| | Synthetic | T255 PE/PET 30% core, 1.5 dtex, 6mm | 8.00 |
| | Binder | Wacker Vinnapas 192 w/ 0.8% Aerosol OT 75 | 4.00 |
| | | Total | 175.00 |

Structure I was a nonwoven substrate including a multi-layer core structure to provide permanent storage with the use of superabsorbent polymer (SAP). Structure I was formed on a Danweb airlaid pilot line. The first layer included 62 gsm of Eucalyptus pulp (Suzano, untreated) mixed with 8 gsm bicomponent fibers (Trevira PE/PET 30% core, 1.5 dtex, 6mm) and deposited on a forming wire. The next layer included 20 gsm starch-based SAP 1, K-Boost (XGF-450, manufactured by Como Cascades LLC, Beavertown, OR) provided on top of the previous layer. The next layer included 54 gsm of cellulose fibers (Georgia-Pacific, GP 4723, fully treated pulp) blended with 21 gsm bicomponent fibers (Trevira, PE/PET 70% core, 1.7 dtex, 6mm). The top and bottom layers were sprayed with 5 gsm (by dry weight) of a polymeric binder in the form of an emulsion (Vinnapas 192, Wacker with 0.8% Aerosol OT 75 surfactant). The basis weight of Structure I was 175 gsm. Structure I included about 11.5% SAP.

The composition of Structure I is provided in Table 6.

**Table 6. Structure I Composition**

| Layer | Type of Material | Raw Materials | Basis Weight (gsm) |
|---|---|---|---|
| | Binder | Wacker Vinnapas 192 w/ 0.8% Aerosol OT 75 | 5.00 |
| 1 | Pulp | Fully-Treated GP 4723 | 54.00 |
| | Synthetic | T255 PE/PET 70% Core, 1.7 dtex, 6mm | 21.00 |
| 2 | SAP | Starch-based SAP 1, K-Boost (XGF-450) | 20.00 |
| 3 | Pulp | Eucalyptus (Suzano, untreated) | 62.00 |
| | Synthetic | T255 PE/PET 30% core, 1.5 dtex, 6mm | 8.00 |
| | Binder | Wacker Vinnapas 192 w/ 0.8% Aerosol OT 75 | 5.00 |
| | | Total | 175.00 |

Structure J was a nonwoven substrate including a multi-layer core structure to provide permanent storage with the use of superabsorbent polymer (SAP). Structure J was formed on a Danweb airlaid pilot line. The first layer included 62 gsm of Eucalyptus pulp (Suzano, untreated) mixed with 8 gsm bicomponent fibers (Trevira PE/PET 30% core, 1.5 dtex, 6mm) and deposited on a forming wire. The next layer included 10 gsm starch-based SAP 2, K-Boost (XGF 463, manufactured by Como Cascades LLC, Beavertown, OR) provided on top of the previous layer. The next layer included 62 gsm of cellulose fibers (Georgia-Pacific, GP 4723, fully treated pulp) blended with 25 gsm bicomponent fibers (Trevira, PE/PET 70% core, 1.7 dtex, 6mm). The top and bottom layers were sprayed with 4 gsm (by dry weight) of a polymeric binder in the form of an emulsion (Vinnapas 192, Wacker with 0.8% Aerosol OT 75 surfactant). The basis weight of Structure J was 175 gsm. Structure J included about 5.7% SAP.

The composition of Structure J is shown in Table 7.

**Table 7. Structure J Composition**

| Layer | Type of Material | Raw Materials | Basis Weight (gsm) |
|---|---|---|---|
| | Binder | Wacker Vinnapas 192 w/ 0.8% Aerosol OT 75 | 4.00 |
| 1 | Pulp | Fully-Treated GP 4723 | 62.00 |
| | Synthetic | T255 PE/PET 70% Core, 1.7 dtex, 6mm | 25.00 |
| 2 | SAP | Starch-based SAP 2, K-Boost (XGF 463) | 10.00 |
| 3 | Pulp | Eucalyptus (Suzano, untreated) | 62.00 |
| | Synthetic | T255 PE/PET 30% core, 1.5 dtex, 6mm | 8.00 |
| | Binder | Wacker Vinnapas 192 w/ 0.8% Aerosol OT 75 | 4.00 |
| | | Total | 175.00 |

Structure K was a nonwoven substrate including a multi-layer core structure to provide permanent storage with the use of superabsorbent polymer (SAP). Structure K was formed on a Danweb airlaid pilot line. The first layer included 62 gsm of Eucalyptus pulp (Suzano, untreated) mixed with 8 gsm bicomponent fibers (Trevira, PE/PET 30% core, 1.5 dtex, 6mm) and deposited on a forming wire. The next layer included 20 gsm starch-based SAP 2, K-Boost (XGF 463, manufactured by Como Cascades LLC, Beavertown, OR) provided on top of the previous layer. The next layer included 54 gsm of cellulose fibers (Georgia-Pacific, GP 4723, fully treated pulp) blended with 21 gsm bicomponent fibers (Trevira, PE/PET 70% core, 1.7 dtex, 6mm). The top and bottom layers were sprayed with 5 gsm (by dry weight) of a polymeric binder in the form of an emulsion (Vinnapas 192, Wacker with 0.8% Aerosol OT 75 surfactant). The basis weight of Structure K was 175 gsm. Structure K included about 11.5% SAP.

The composition of Structure K is provided in Table 8.

**Table 8. Structure K Composition**

| Layer | Type of Material | Raw Materials | Basis Weight (gsm) |
|---|---|---|---|
| | Binder | Wacker Vinnapas 192 w/ 0.8% Aerosol OT 75 | 5.00 |
| 1 | Pulp | Fully-Treated GP 4723 | 54.00 |
| | Synthetic | T255 PE/PET 70% Core, 1.7 dtex, 6mm | 21.00 |
| 2 | SAP | Starch-based SAP 2, K-Boost (XGF 463) | 20.00 |
| 3 | Pulp | Eucalyptus (Suzano, untreated) | 62.00 |
| | Synthetic | T255 PE/PET 30% core, 1.5 dtex, 6mm | 8.00 |
| | Binder | Wacker Vinnapas 192 w/ 0.8% Aerosol OT 75 | 5.00 |
| | | Total | 175.00 |

### EXAMPLE 2: Feminine Hygiene Napkin Application - Effective Acquisition Time, Rewet, Retention Before Leak, and Wicking (Structures B-D, F, and H-K)

Structures B-D, F, and H-K were tested as an absorbent core in combination with a commercial acquisition distribution layer (Structure G) in feminine hygiene napkin applications for effective acquisition time, rewet, retention before leak, and wicking liquid characteristics as compared to commercial absorbent cores (Structures A and E).

### Sample Preparation

Structure A was a control sample of a commercially available absorbent core. Structure A was a 175 gsm commercial multi-bonded airlaid (MBAL)(175 gsm, Vizorb 3950) including about 20% SAP (Georgia-Pacific, Steinfurt, Germany).

Structure E was a control sample of an absorbent core removed from a commercially available product. Structure E was a 200 gsm hydrogen bonded airlaid (HBAL) core that included about 30% SAP. Structure E was tested at 6.3 x 2.2 inches (which was smaller than other samples).

Structure G was a commercially available acquisition distribution layer. Structure G was a 34 gsm carded web (Shalag Nonwovens, TABCW, product code STACT8H34) including polyester fibers. Structure G provided an ADL component of absorbent systems tested. Each of the tested composites or articles contained a top layer which was Structure G and a bottom layer.

### Liquid Acquisition Time and Rewet Testing

The liquid acquisition characteristics of each sample were measured with a synthetic blood solution. Synthetic blood from Johnson, Moen & Co., Inc. (Rochester, MN) (Lot # 201141; February 2014) was used. The synthetic blood had a surface tension of 40-44 mN/m (ASTM F23.40-F1670), viscosity of 3.020-7.700 mPas, and included various chemicals including ammonium polyacrylate polymer, Azo Red Dye, and HPLC distilled water, among other ingredients. The synthetic blood was used "as-is" without any dilution.

The testing apparatus included a 29.2 cm x 19.1 cm x 0.6 cm hard plastic plate with a 1.9 cm inner diameter hole cut in the center. Attached above the hole was a weighted stainless-steel cylinder with a 1.9 cm inner diameter. The cylinder had a height of 5.1cm, which made the complete apparatus a total of 5.7 cm tall and weighing a total of 747.3 g.

The prepared composite or article (6.5 cm x 20.5 cm or 6.5 cm x 21.5 cm) including a sample in combination with an acquisition distribution layer was compressed at 4 bars compaction in a roller press to simulate the stress of the converting process. The composite or article was insulted with 4 mL of synthetic blood, depending on the test performed, at a rate of 10 mL/min. The acquisition time was measured from the start of insult until the synthetic blood was no longer visible in the insult cylinder. A total of three insults were performed, yielding acquisition times, #1, #2, and #3. The time interval between the insults was 10 minutes. The obtained results of the acquisition times were used then to calculate the Effective Acquisition Times (EAT). The EAT values are calculated as the differences between the total Acquisition Times (TAT) and 24 seconds, which is the Insult Time (IT) (i.e., the time during which all the liquid (in this case 4 mL) is delivered onto the tested absorbent system).

After measuring the three acquisition times, the rewet characteristics of each material were analyzed. After the third acquisition time measurement, three pre-weighed square plies (10.1 cm x 10.1 cm) of collagen (Coffi collagen, Viscofan USA) were placed on top of the tested composite or article. A thin plexiglass plate and a weight were placed on top of the collagen plies for 1 minute. The plexiglass and the weight exerted a total pressure of 1.7 kPa. The collagen plies were weighed to determine the rewet result. The results of each test were averages of three measurements.

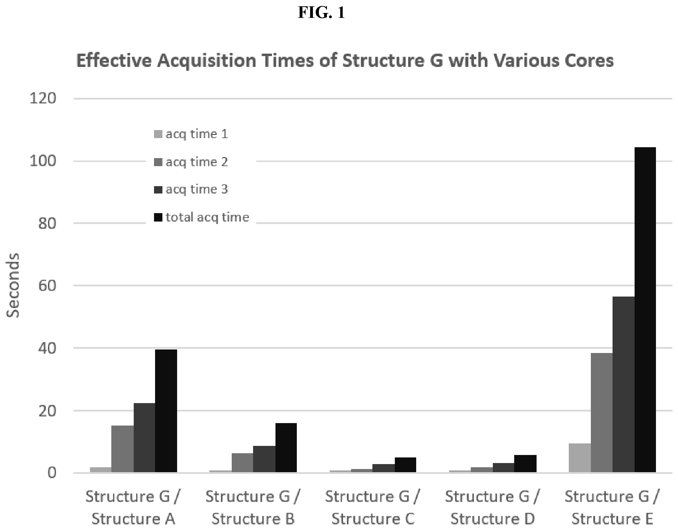

The test results are provided in FIGS. 1-4.

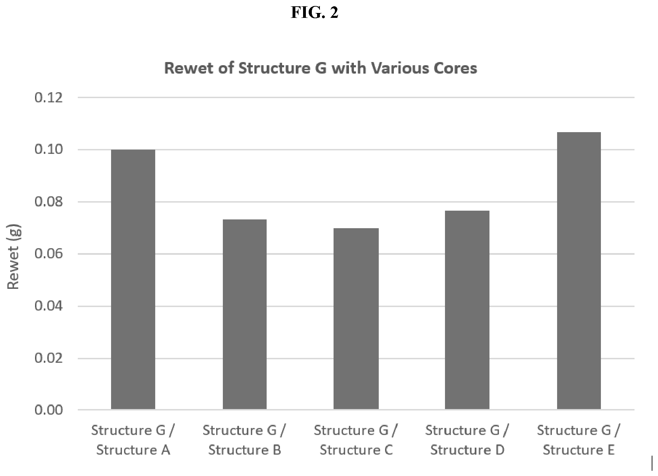

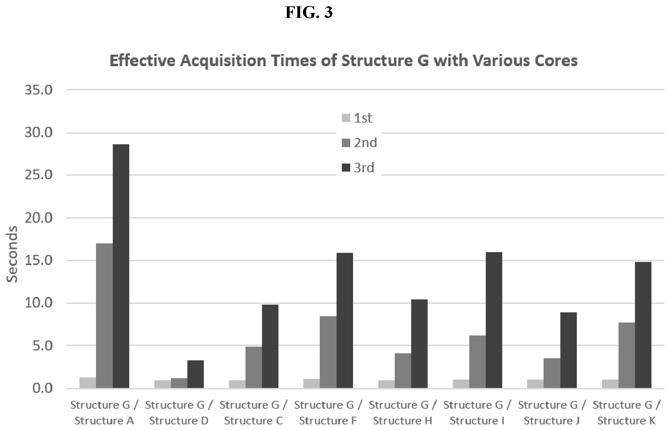

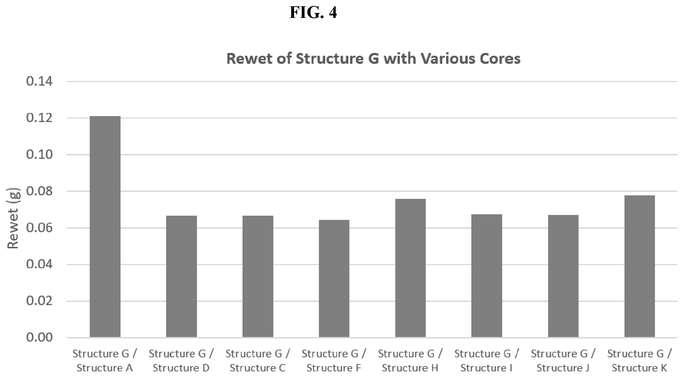

FIGS. 1 and 2 provide the Effective Acquisition Time and rewet for Structures B, C and D as compared to commercially available cores (Structures A and E), respectively. FIGS. 3 and 4 provide Structures B, C, and D have increased performance than either of the commercially available cores (Structures A and E). Without being bound to a particular theory, it is hypothesized that the bottom layers of Structures B, C and D have higher capillary pressure than the top layers of these structures and can distribute the liquid efficiently from the insult point thus leaving enough void volume to accept more liquid and thus allowing for better utilization of the absorbent core. High capillary pressure of these layers can also allow for holding the liquid away from the surface of the core and of the acquisition distribution layer component of the absorbent systems with the experimental core (Structures B, C and D). This results in lower rewet as shown in FIG. 2.

As provided in FIG. 1, liquid can be acquired by the absorbent systems with the experimental cores (Structures B, C and D) significantly faster than absorbent systems including commercial cores (Structures A and E). This results in shorter Effective Acquisition Time as shown in FIG. 1. The results in FIG. 1 provide that absorbent systems with experimental cores (Structures B, C and D) having relatively low amounts or no SAP (respectively, about 11.5%, about 5.7% and 0%) compared to the commercial control absorbent cores (20% and 30% SAP) having a similar or higher total basis weight had increased performance. Structures B, C and D exhibited improved performance while including a lower amount of synthetic components and being more economical than conventional absorbent cores.

FIGS. 3 and 4 provide the Effective Acquisition Time and rewet for Structures C, D, F, H, I, J and K as compared to commercially available cores (Structure A), respectively. FIGS. 3 and 4 provide Structures C, D, F, H, I, J and K had increased performance than the commercially available core (Structures A). Without being bound to theory, it is hypothesized that the bottom layers of Structures C, D, F, H, I, J and K have higher capillary pressure than the top layers of these structures and can distribute the liquid efficiently from the insult point thus leaving enough void volume to accept more liquid and thus allow for better utilization of the absorbent core. High capillary pressure of these layers can also allow for holding the liquid away from the surface of the core and of the acquisition distribution layer of the component of the absorbent systems with the experimental core (Structures C, D, F, H, I, J and K). Thus provides for lower rewet as shown in FIG. 4.

The results in FIG. 3 provide that liquid can be acquired by the absorbent systems with the experimental cores (Structures C, D, F, H, I, J and K) significantly faster than an absorbent system including the commercial core (Structure A). The results in FIG. 3 provide that absorbent systems with experimental cores (Structures C, D, F, H, I, J and K) having relatively low amounts or no SAP compared to the commercial control absorbent core (20% SAP) and having a similar basis weight. Structures C, D, F, H, I, J had increased performance while including a lower amount of synthetic components and being more economical than the conventional absorbent cores.

### Retention Before Leak and Wicking Distance Testing

Samples were also tested for their fluid retention and distribution properties. The liquid acquisition characteristics of each structure were measured with a synthetic blood solution to test retention before leak and wicking distance. Synthetic blood from Johnson, Moen & Co., Inc. (Rochester, MN) (Lot #201141; February 2014) was used. The synthetic blood had a surface tension of 40-44 mN/m (ASTM F23.40-F1670), viscosity of 3.020-7.700 mPas, and included various chemicals including ammonium polyacrylate polymer, Azo Red Dye, and HPLC distilled water, among other ingredients.

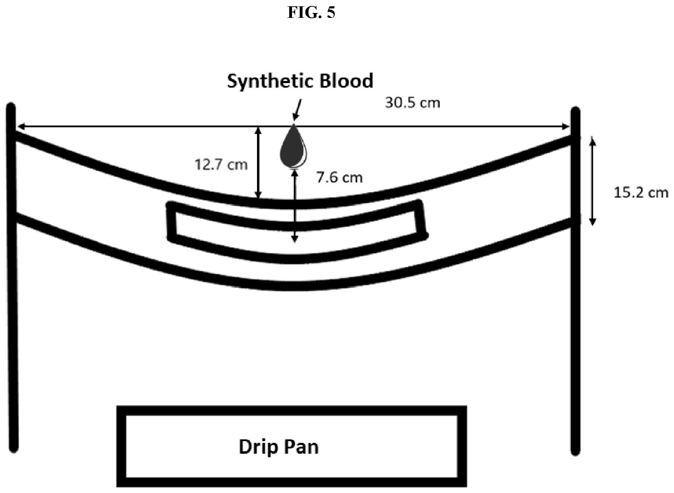

The testing apparatus included a mesh net (5/8" x 5/8" mesh) draped over a testing stand that measured 30.5 cm x 15.2 cm. The mesh net was arranged to provide a 12.7 cm drop in the center when compared to level. The synthetic blood was dosed with a peristaltic pump at a rate of 10mL/min and was allowed to drop 7.6 cm before landing on the tested substrate. A drip pan was provided below the mesh net directly under the sample to catch the synthetic blood and allow for an endpoint to the test. A schematic representation of the testing apparatus is provided in FIG. 5.

The samples were cut to a standard 20.3 cm x 6.4 cm and were measured for initial thickness and basis weight. If the samples had a back sheet and have gone through the converting process, the samples were directly tested. Samples that did not have a back sheet had a 0.7 mm plastic sheet glued to the bottom of the sample. The plastic sheet was cut to the same size as the sample (20.3 cm x 6.4 cm). Glue was applied to the bottom of the sample lengthwise in two lines, approximately 1.3 cm from each long edge. The 0.7 mm plastic sheet was then applied to the back of the sample. The plastic sheet was pressed down into the glue gently to avoid altering the acquisition traits before compression.

The samples that had not been converted were compressed using a roller press with 4 bars of pressure to simulate the pressures of converting. Once the samples were compressed, the thickness measurements of the samples were taken, and the samples were tested. The peristaltic pump was set to 10mL/minute and the output was verified using a 10 mL graduated cylinder and timer. A drip pan was placed under the mesh net to receive any leakage.

The sample was placed with the back sheet down in the center of the mesh net. The long side of the sample was parallel to the long side of the mesh net. The peristaltic pump line was set to drip the synthetic blood directly into the middle of the sample from 3 inches above the sample. The peristaltic pump was turned on and the timer was started as soon as the first drop of synthetic blood landed on the sample. The timer was stopped as soon as the first drop of synthetic blood dripped from the sample into the drip pan. The peristaltic pump was turned off, the time was recorded in seconds, and the wicking distance was measured. The wicking distance was measured on the bottom of the sample and was a measure of liquid distribution (mm). The results of each test were of three measurements.

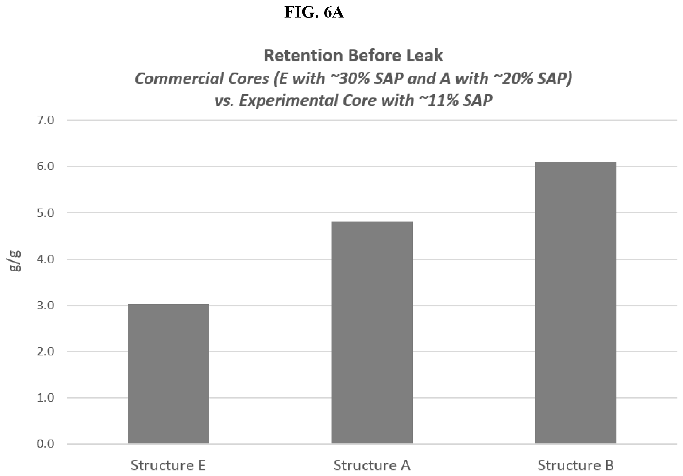

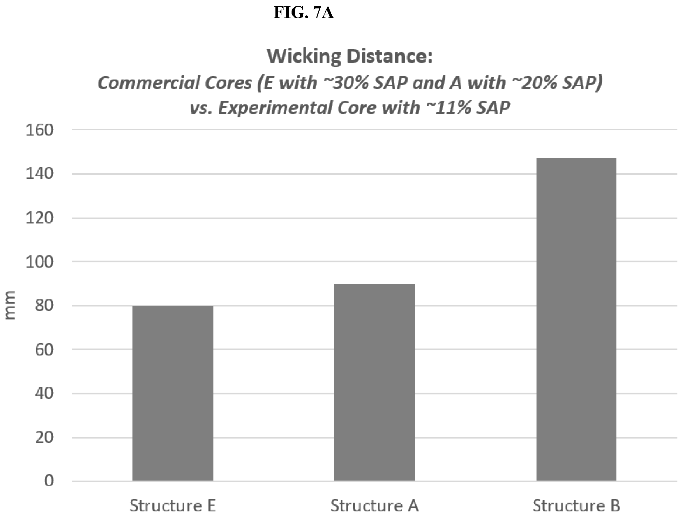

The test results are provided in FIGS. 6A-C and 7A-C.

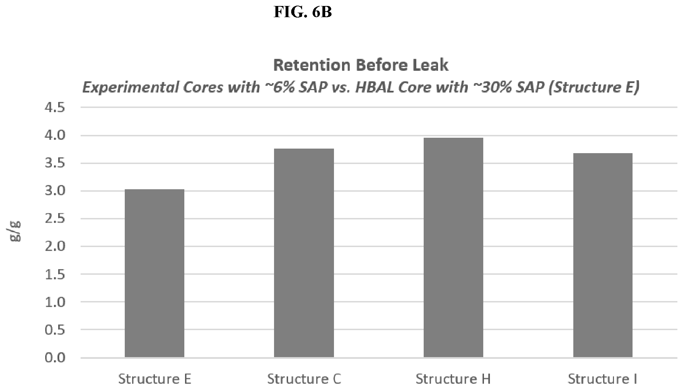

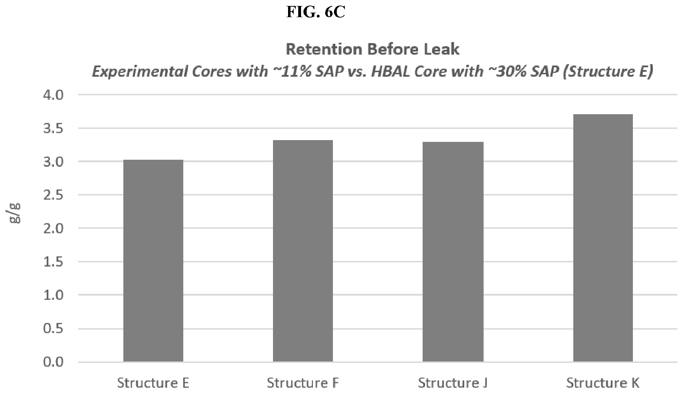

FIGS. 6A-C provide the retention before leak test results as compared to commercial cores. FIG. 6A provides the retention before leak results of Structure B. FIG. 6B provides the retention before leak results of Structures C, H and I. FIG. 6C provides the retention before leak results of Structures F, J, and K. The data FIGS. 6A-C provide that the experimental Structures B, C, D, F, H, I, J and K have increased performance than either of the commercially available cores (Structures A and E). Without being bound to theory, it is hypothesized that the bottom layers of the experimental Structures have higher capillary pressure than the top layers of these structures and can distribute the liquid efficiently from the insult point thus leaving enough void volume to accept more liquid and thus allow for better utilization of the absorbent core. High capillary pressure of these layers can also allow for holding the liquid away from the surface of the core. The results in FIGS. 6A-C suggest that more liquid can be acquired by the experimental core. The results in FIGS. 6A-C further suggest that the experimental core can provide structures that can retain more liquid before leakage occurs as compared to absorbent systems including commercial cores (Structures A and E). Structures B, C, D, F, H, I, J having relatively low amounts of SAP as compared to the commercial control absorbent cores provided increased performance and included lower amount of synthetic components while being more economical than conventional absorbent cores. In particular, Structures H, I, J and K each include a very low content of synthetic components because these Structures each contain biodegradable, starch-based SAP.

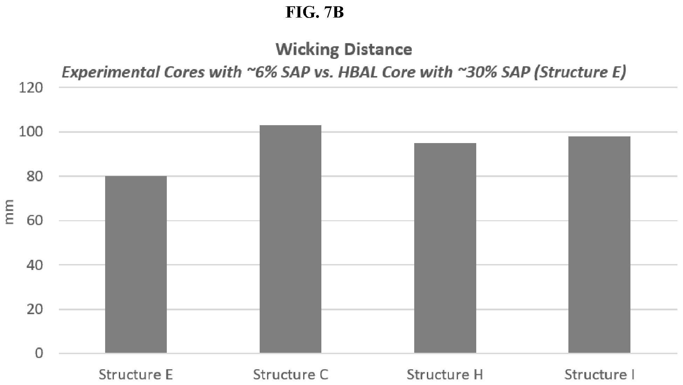

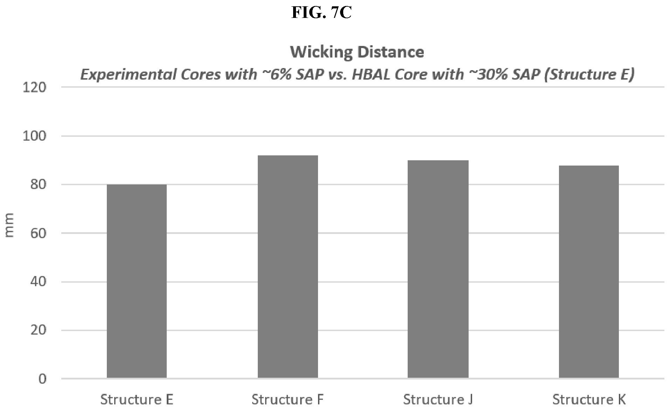

FIGS. 7A-C provide the wicking test results as compared to commercial cores (Structures A and E). FIG. 7A provides the wicking test results of Structure B. FIG. 7B provides the wicking tests results of Structures C, H and I. FIG. 7C provides the wicking test results of Structures F, J and K. The data in FIGS. 7A-C provide that the experimental Structures B, C, F, H, I, J and K have increased performance than either of the commercially available cores (Structures A and E). Without being bound to theory, it is hypothesized that the bottom layers of the experimental Structures have higher capillary pressure than the top layers of these structures and can distribute the liquid efficiently from the insult point thus leaving enough void volume to accept more liquid and thus allow for better utilization of the absorbent core. High capillary pressure of these layers allows also for holding the liquid away from the surface of the core. The results in FIGS. 7A-C suggest that the liquid can be distributed in the experimental core further from the insult point than in the case of the systems containing commercial cores (Structures A and E).

### EXAMPLE 3: Hybrid Absorbent Structures

The present Example provides hybrid absorbent structures that can provide integrated acquisition, distribution and storage functions in one unitary structure. The hybrid absorbent structures can be used as acquisition distribution layers, for example, as a component of personal hygiene products.

Structure 1A was a unitary, two-sided hybrid nonwoven material. When used as an acquisition distribution layer (ADL), the top layer included a synthetic nonwoven component and the bottom layer included cellulose fibers bonded with a latex binder. Structure 1A was formed using a lab padformer. Structure 1A was formed by laying cellulose fibers (Georgia-Pacific, GP 4723, fully-treated pulp, Leaf River) in an amount of 18.0 gsm on a layer of 34 gsm through-air bonded carded nonwoven web (Shalag Nonwovens, product code STACT8H34). The cellulosic fiber layer was sprayed with 3.0 gsm of a polymeric binder (based on its dry weight) in the form of an emulsion (Vinnapas 192, Wacker, 15%) with 0.20 gsm of a surfactant (Aerosol OT 75, Cytec Industries). Structure 1A was cured in an oven (145 °C, 4 min) to bond the cellulose fibers and the carded nonwoven together. The total basis weight of Structure 1A was 55 gsm.

The composition of Structure 1A is shown in Table 9.

**Table 9. Structure 1A Composition**

| Layer | Type of Material | Raw Materials | Basis Weight (gsm) |
|---|---|---|---|
| 1 | Carded Web | Shalag Nonwovens, Carded Nonwoven Web, Product Code STACT8H34 (TABCW) | 34.00 |
| 2 | Pulp | Leaf River Fully-Treated GP 4723 | 18.00 |
| | Binder | Wacker Vinnapas 192 w/ 0.2 Aerosol OT 75 | 3.00 |
| | | Total | 55.00 |

Structure 1B was a unitary, two-sided hybrid nonwoven material. When used as an acquisition distribution layer (ADL), the top layer included a synthetic nonwoven component and the bottom layer included cellulose fibers bonded with a latex binder. Structure 1B was formed using a lab padformer. Structure 1B was formed by laying cellulose fibers (Georgia-Pacific, GP 4723, fully-treated pulp, Leaf River) in an amount of 14.0 gsm on a layer of 34 gsm through-air bonded carded nonwoven web (Shalag Nonwovens, product code STACT8H34). The cellulosic fiber layer was sprayed with 2.0 gsm of a polymeric binder (based on its dry weight) in the form of an emulsion (Vinnapas 192, Wacker, 15%) with 0.20 gsm of a surfactant (Aerosol OT 75, Cytec Industries). Structure 1B was cured in an oven (145 °C, 4 min) to bond the cellulose fibers and the carded nonwoven together. The total basis weight of Structure 1B was 50 gsm.

The composition of Structure 1B is shown in Table 10.

**Table 10. Structure 1B Composition**

| Layer | Type of Material | Raw Materials | Basis Weight (gsm) |
|---|---|---|---|
| 1 | Carded Web | Shalag Nonwovens, Carded Nonwoven Web, Product Code STACT8H34 (TABCW) | 34.00 |
| 2 | Pulp | Leaf River Fully-Treated GP 4723 | 14.00 |
| | Binder | Wacker Vinnapas 192 w/ 0.2 Aerosol OT 75 | 2.00 |
| | | Total | 50.00 |

Structure 2 was a multi-layer unitary structure formed on a Danweb airlaid pilot line. Structure 2 included a synthetic fiber layer, directionally layered to allow for acquisition and rewet, and a multi-layer core structure to provide permanent liquid storage and distribution. A carded nonwoven (Shalag Nonwovens, product code STACT8H34) was used as a carrier for the airlaid nonwoven structure. The first layer included 69 gsm of cellulose fibers (Georgia-Pacific, GP 4723, fully treated pulp, Leaf River) blended with 26 gsm bicomponent fibers (Trevira, 1.7 dtex, 6 mm). The bicomponent fibers included a polyethylene (PE) sheath (30%) and polyethylene terephthalate (PET) core (70%). The top layer included 62 gsm of Eucalyptus pulp (Suzano, untreated) mixed with 8 gsm of bicomponent fibers (Trevira, 1.5 dtex, 6mm). The bicomponent fibers included a PE sheath (70%) and PET core (30%). The Eucalyptus layer was also sprayed with 5 gsm (based on dry weight) of a polymeric binder in the form of an emulsion (Vinnapas 192, Wacker with 0.8% Aerosol OT 75 surfactant). The total basis weight of Structure 2 was 200 gsm.

The composition of Structure 2 is shown in Table 11.

**Table 11. Structure 2 Composition**

| Layer | Type of Material | Raw Materials | Basis Weight (gsm) |
|---|---|---|---|
| 1 | Carded Web | Shalag Nonwovens, Carded Nonwoven Web, Product Code STACT8H34 (TABCW) | 30.00 |
| 2 | Pulp | Leaf River Fully-Treated GP 4723 | 69.00 |
| | Synthetic Fiber | Trevira, 1.7 dtex, 6mm (30% PE / 70% PET Core) | 26.00 |
| 3 | Pulp | Eucalyptus (Suzano, untreated) | 62.00 |
| | Synthetic Fiber | Trevira, 1.5 dtex, 6mm (70% PE / 30% PET Core) | 8.00 |
| | Binder | Wacker Vinnapas 192 w/ 0.8% Aerosol OT 75 | 5.00 |
| | | Total | 200.00 |

Structure 3 was a multi-layer unitary structure formed on a Danweb airlaid pilot line. Structure 3 included a synthetic fiber layer, directionally layered to allow for acquisition and rewet, and a multi-layer core structure to provide permanent liquid storage with the use of superabsorbent polymer (SAP) and liquid distribution. A carded nonwoven (Shalag Nonwovens, product code STACT8H34) was used as a carrier for the airlaid nonwoven structure. The first layer included 62 gsm of cellulose fibers (Georgia-Pacific, GP 4723, fully treated pulp, Leaf River) blended with 23 gsm bicomponent fibers (Trevira, 30% PE/70% PET core, 1.7 dtex, 6mm). The next layer included 10 gsm of superabsorbent polymer (SAP) (Evonik Favor SXM 7900). The top layer included 62 gsm of Eucalyptus pulp (Suzano, untreated) mixed with 8 gsm of bicomponent fibers (Trevira, 70% PE/30% PET core, 1.5 dtex, 6 mm). The Eucalyptus layer was sprayed with 5 gsm (by dry weight) of a polymeric binder in the form of an emulsion (Vinnapas 192, Wacker with 0.8% Aerosol OT 75 surfactant). The total basis weight of Structure 3 was 200 gsm.

The composition of Structure 3 is shown in Table 12.

**Table 12. Structure 3 Composition**

| Layer | Type of Material | Raw Materials | Basis Weight (gsm) |
|---|---|---|---|
| 1 | Carded Web | Shalag Nonwovens, Carded Nonwoven Web, Product Code STACT8H34 (TABCW) | 30.00 |
| 2 | Pulp | Leaf River Fully-Treated GP 4723 | 62.00 |
| | Synthetic Fiber | Trevira, 1.7 dtex, 6mm (30% PE / 70% PET Core) | 23.00 |
| 3 | SAP | Evonik Favor SXM 7900 | 10.00 |
| 4 | Pulp | Eucalyptus (Suzano, untreated) | 62.00 |
| | Synthetic Fiber | Trevira, 1.5 dtex, 6mm (70% PE / 30% PET core) | 8.00 |
| | Binder | Wacker Vinnapas 192 w/ 0.8% Aerosol OT 75 | 5.00 |
| | | Total | 200.00 |

Structure 4 was a multi-layer unitary structure formed on a Danweb airlaid pilot line. Structure 4 included a synthetic fiber layer, directionally layered to allow for acquisition and rewet, and a multi-layer core structure to provide permanent storage with the use of superabsorbent polymer (SAP) and wicking. A carded nonwoven (Shalag Nonwovens, product code STACT8H34) was used as a carrier for the airlaid nonwoven structure. The first layer included 54 gsm of cellulose fibers (Georgia-Pacific, GP 4723, fully treated pulp, Leaf River) blended with 21 gsm of bicomponent fibers (Trevira, 30% PE/70% PET core, 1.7 dtex, 6 mm). The next layer included 20 gsm of superabsorbent polymer (SAP) (Evonik Favor SXM 7900). The top layer included 62 gsm of Eucalyptus pulp (Suzano, untreated) mixed with 8 gsm of bicomponent fibers (Trevira, 70% PE/30% PET core, 1.5 dtex, 6 mm). The Eucalyptus layer was sprayed with 5 gsm (by dry weight) polymeric binder in the form of an emulsion (Vinnapas 192, Wacker with 0.8% Aerosol OT 75 surfactant). The total basis weight of Structure 4 was 200 gsm.

The composition of Structure 4 is shown in Table 13.

**Table 13. Structure 4 Composition**

| Layer | Type of Material | Raw Materials | Basis Weight (gsm) |
|---|---|---|---|
| 1 | Carded Web | Shalag Nonwovens, Carded Nonwoven Web, Product Code STACT8H34 (TABCW) | 30.00 |
| 2 | Pulp | Leaf River Fully-Treated GP 4723 | 54.00 |
| | Synthetic Fiber | Trevira, 1.7 dtex, 6mm (30% PE / 70% PET Core) | 21.00 |
| 3 | SAP | Evonik Favor SXM 7900 | 20.00 |
| 4 | Pulp | Eucalyptus (Suzano, untreated) | 62.00 |
| | Synthetic Fiber | Trevira, 1.5 dtex, 6mm (70% PE / 30% PET core) | 8.00 |
| | Binder | Wacker Vinnapas 192 w/ 0.8% Aerosol OT 75 | 5.00 |
| | | Total | 200.00 |

### EXAMPLE 4: Feminine Hygiene Napkin Application - Liquid Acquisition Time and Rewet Testing (Structure 1A)

Structure 1A was tested as an acquisition distribution layer in an absorbent system including a commercial absorbent core in feminine hygiene napkin applications for liquid acquisition and rewet characteristics as compared to commercial absorbent systems.

### Sample Preparation

Structure 1A (20.3 cm x 6.4 cm) was placed on top of a 175 gsm commercial core (Vizorb 3950, Georgia-Pacific, Steinfurt) (20.3 cm x 6.4 cm) to provide an absorbent system. The resultant absorbent system was roller-pressed at 4 bars of pressure. Control 1 was a commercial 60 gsm latex-bonded airlaid (LBAL). Control 2 was a commercial 95 gsm multi-bonded airlaid (MBAL). Control 3 was a commercial 34 gsm carded nonwoven (Shalag Nonwovens, Product Code STACT8H34). Control 1, Control 2, and Control 3 were each separately placed on top of a 175 gsm commercial core (Vizorb 3950, Georgia-Pacific, Steinfurt) (20.3 cm x 6.4 cm) to provide an absorbent system. Each control was tested as an acquisition distribution layer and was placed on top of a 175 gsm commercial core (Vizorb 3950, Georgia-Pacific, Steinfurt) (20.3 cm x 6.4 cm) to provide an absorbent system. The resultant absorbent system was roller-pressed at 4 bars of pressure.

### Liquid Acquisition Time and Rewet Testing

The liquid acquisition time and rewet of the absorbent systems including Control 1, Control 2, and Control 3 were evaluated in the same manner as the absorbent system including Structure 1A. The testing device included a plexiglass plate with a stainless-steel cylinder attached (748 g, inner diameter 2.2 cm) and was placed on top of the absorbent system for testing. The absorbent system was insulted with 7mL of synthetic blood (Johnson, Moen & Co., Inc., ASTM F1670 Synthetic Blood, viscosity 5.56 mPa/s, surface tension 40-44 mN/m) delivered to the topside of the absorbent system at rate of 10mL/min using a mini-pump (Fisher Scientific). The mini-pump and a timer were activated simultaneously, and the absorbent system was insulted for 42 seconds. The Total Acquisition Time #1 (TAT#1) was measured from when the dosing of the synthetic blood began until the synthetic blood was no longer seen inside the stainless-steel cylinder. A total of three Total Acquisition Times (TAT #1, TAT #2, and TAT #3) were measured with 10-minute idle time between the beginning of the previous insult and the beginning of the next insult. The absorbent system was left to rest for another 10 minutes after the third insult. Afterwards, four pieces of Coffi collagen sheets (Viscofan, 17.8 cm x 10 cm) were placed on top of the testing device. A thin plexiglass plate and a weight were placed on top of the Coffi sheets for 1 minute. The plexiglass plate and the weight exerted a total of 0.86 kPa on the 20.3 cm x 6.4 cm area. Afterwards, the Coffi collagen sheets were weighed to determine the rewet. The rewet was a difference between the weights of the Coffi collagen sheets after and before the test. The acquisition time and rewet results were averages of three measurements.

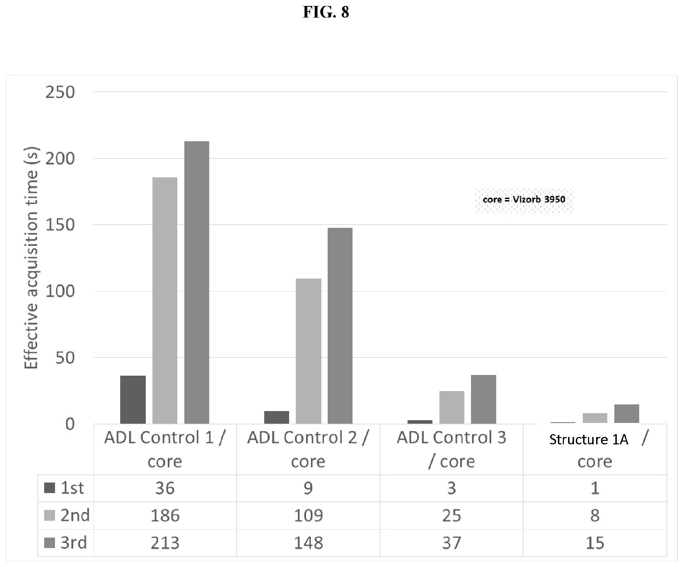

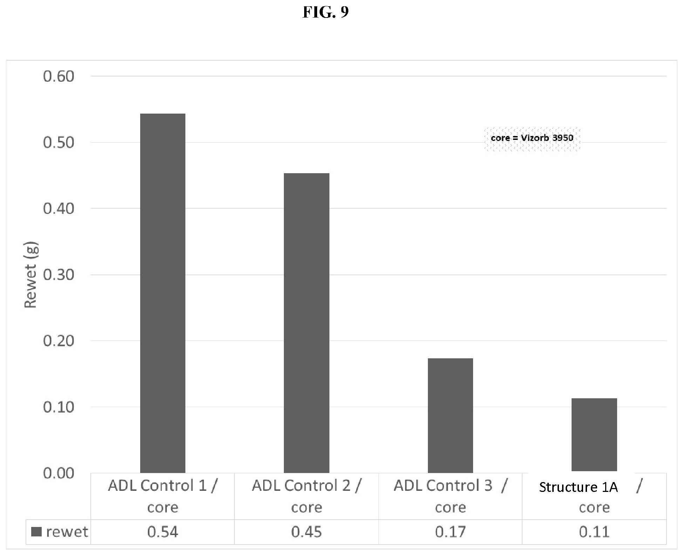

The test results are provided in FIGS. 8 and 9 and Tables 14 and 15.

FIG. 8 is a graphic representation of the results of the average Effective Acquisition Time (EAT) measurements. The EAT values were calculated as the differences in the Total Acquisition Times (TAT) and 42 seconds, which was the Insult Time (IT) (i.e., the time during which all the liquid in the stainless-steel cylinder (7mL) was delivered onto the tested absorbent system). The Effective Acquisition Time (EAT) was calculated as the Total Acquisition Times (TAT) minus the Insult Time (IT). The insult time was how long the pump was turned on to deliver the synthetic blood.$\left(EAT\right) = \left(TAT\right) - \left(IT\right)$

As shown in FIG. 8 and Table 14, absorbent systems including Structure 1A as an acquisition distribution layer had higher liquid acquisition performance than all control acquisition distribution commercial nonwoven materials.

**Table 14. Effective Acquisition Time (EAT) (s)**

| | Control 1/Core | Control 2/Core | Control 3/Core | Structure 1A/Core |
|---|---|---|---|---|
| 1 | 36 | 9 | 3 | 1 |
| 2 | 186 | 109 | 25 | 8 |
| 3 | 213 | 148 | 37 | 15 |

FIG. 9 is a graphic representation of the results of the rewet testing. As shown in FIG. 9 and Table 15, absorbent systems including Structure 1A as an acquisition distribution layer can provide more comfort to the end user in terms of dryness than all control acquisition distribution commercial nonwoven materials. Overall, absorbent systems including Structure 1 had the lowest rewet at 0.11 g.

**Table 15. Rewet**

| | Control 1/Core | Control 2/Core | Control 3/Core | Structure 1A/Core |
|---|---|---|---|---|
| Rewet (g) | 0.54 | 0.45 | 0.17 | 0.11 |

### EXAMPLE 5: Diaper Application - Rewet Testing (Structure 1B)

Structure 1B was tested as an acquisition distribution layer in a diaper application for rewet characteristics as compared to commercial diaper products.

### Sample Preparation

A commercial diaper containing a 50 gsm carded nonwoven acquisition distribution layer was used as a Control diaper and tested for rewet. The elastic bands around the entire Control diaper were removed. The original 50 gsm carded nonwoven acquisition distribution layer was kept intact inside the diaper. The Control diaper was tested without any further compaction since the diaper underwent compaction during the manufacturing process.

The same commercial diaper was deconstructed. The original 50 gsm carded nonwoven acquisition distribution layer was removed and replaced with Structure 1B (19.0 cm x 8.0 cm). The diaper's nonwoven topsheet was placed back on top of the diaper. Afterwards, the diaper was compressed with 4 bars of pressure via a roller press.

### Rewet Testing

The rewet of the diaper with Structure 1B as an acquisition distribution layer was evaluated in the same manner as the Control diaper. Testing equipment was placed onto the diaper. The testing equipment exerted approx. 2.8 kPa onto the diaper. The testing equipment included an attached saline delivery cylinder (inside diameter 3.8 cm). The saline delivery cylinder was placed approx. 2 inches from the acquisition distribution layer's end corresponding to the frontside of the diaper. The Control diaper was insulted with 75 mL of 0.9% sodium chloride (NaCl) solution by turning on a pump that delivered the solution at a rate of 7 mL/sec for 10.7 seconds. Once the liquid was completely absorbed, a 20-minute timer was activated. At 20-minutes, a second 75 mL aliquot of 0.9% sodium chloride (NaCl) solution was used to insult the product. Once the liquid was completely absorbed, the 20-minute timer was activated. At 20-minutes, the testing equipment was removed. Eight pieces of Curity (10 cm x 10 cm nonwoven, All Purpose Sponges, Covidien) were unfolded to increase the coverage area to 20 cm x 10 cm. The eight pieces of Curity were placed on top of the wet diaper, covering the entire 19.0 cm x 8.0 cm acquisition distribution layer. The foam-insult cylinder-weights were placed back on top of the diaper, with the insulting cylinder on the opposite end of the diaper's insulted area. A 5-minute timer was activated. At 5-minutes, the Curity were weighed to calculate the rewet value. The final result of the rewet was an average of the results of three tests.

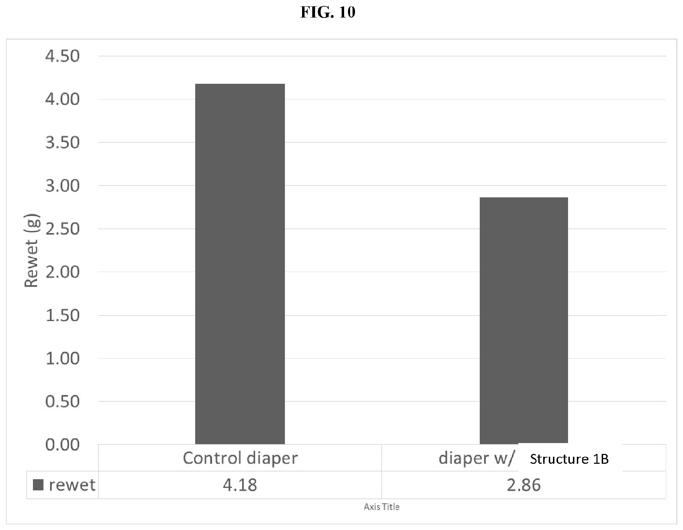

The test results are provided in FIG. 10. FIG. 10 provides the rewet measurements of the Control diaper and the diaper including Structure 1B as an acquisition distribution layer. The Control diaper had a rewet of 4.18 g and the diaper including Structure 1B had a lower rewet value of 2.86 g. A diaper with Structure 1B as an acquisition distribution layer can provide a dryer feel than a diaper with the original carded nonwoven acquisition distribution layer.

### EXAMPLE 6: Feminine Hygiene Napkin Application - Liquid Acquisition Time, Rewet and Wicking Testing (Structures 2-4)

Structures 2-4 were tested for liquid acquisition, rewet and wicking characteristics as compared to absorbent systems of commercial feminine hygiene napkins (Control A-D).

### Sample Characteristics

Control A was a commercial sanitary napkin (Sannap A). The absorbent system had a total basis weight of 325 gsm. The acquisition distribution layer included a TABCW layer having a basis weight of about 46 gsm and an airlaid sheet having a basis weight of about 77 gsm. The core was a hydrogen bonded airlaid sheet including about 30% SAP material. The core had a basis weight of about 200 gsm.

Control B was a commercial sanitary napkin (Sannap B). The absorbent system had a total basis weight of about 206 gsm. The acquisition distribution layer was an airlaid sheet having a basis weight of about 97 gsm. The core was an airlaid sheet containing greater than about 10% SAP material. The core had a basis weight of about 109 gsm.

Control C was a commercial sanitary napkin (Sannap C). The absorbent system had a total basis weight of about 241 gsm. The acquisition distribution layer was an airlaid sheet having a basis weight of about 102 gsm. The core was an airlaid sheet containing greater than about 10% SAP material. The core had a basis weight of about 139 gsm.

Control D was a commercial sanitary napkin (Sannap D). The absorbent system had a total basis weight of about 215 gsm. The spunbound acquisition distribution layer had a basis weight of about 57 gsm. The core was an airlaid sheet containing greater than about 10% SAP material. The core had a basis weight of about 158 gsm.

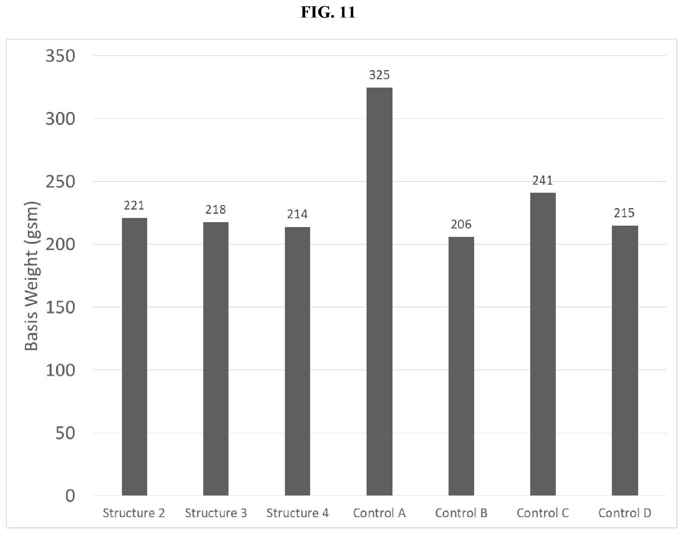

FIG. 11 is a graphic representation of the basis weight of Structures 2-4 as compared to the tested commercial sanitary napkins (Control A-D). Structures 2 and 3 had a lower basis weight than Control A and C. Structure 4 had a lower basis weight than Control A, C and D.

### Liquid Acquisition Time and Rewet Testing

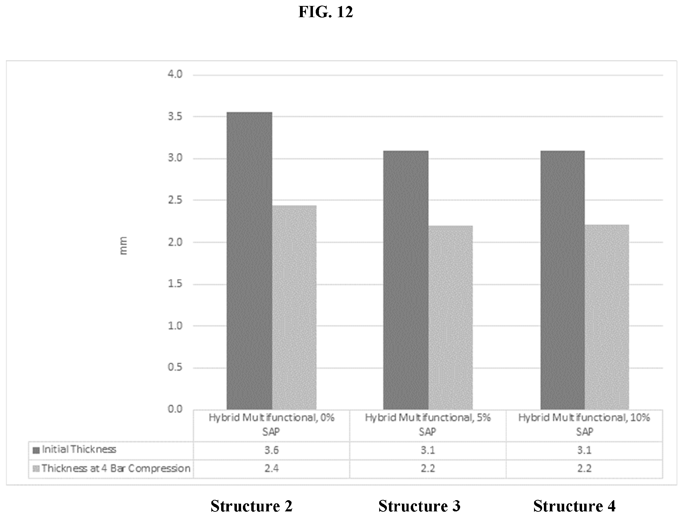

The liquid acquisition time and rewet of Structures 2-4 were evaluated in the same manner as the tested commercial sanitary napkins (Control A-D). The absorbent system (6.5 cm x 20.5 cm or 6.5 cm x 21.5 cm) was roller-pressed at 4 bars of pressure. FIG. 12 and Table 16 provide the thickness of Structures 2-4 before and after 4 bar compression.

**Table 16. Thickness - Structures 2-4**

| | Structure 2 | Structure 3 | Structure 4 |
|---|---|---|---|
| Initial Thickness (mm) | 3.6 | 3.1 | 3.1 |
| Thickness at 4 Bar Compression (mm) | 2.4 | 2.2 | 2.2 |

Testing equipment included a plexiglass plate with a cylinder attached (748 g, inner diameter 2.2 cm) placed on top of the absorbent system. The absorbent system was insulted with 4 mL of synthetic blood (Johnson, Moen & Co., Inc., ASTM F1670 Synthetic Blood, viscosity 5.56 mPa/s, surface tension 40-44 mN/m), delivered to the topside at a rate of 10 mL/min using a mini-pump (Fisher Scientific). The mini-pump and a timer were activated simultaneously, and the absorbent system was insulted for 24 seconds. The Total Acquisition Time #1 (TAT #1) was measured from the moment the dosing of the synthetic blood began until the synthetic blood was no longer seen inside the stainless-steel cylinder. A total of three Total Acquisition Times (TAT #1, TAT #2, and TAT #3) were measured with 10-minute idle time between the beginning of the previous insult and the beginning of the next insult. The absorbent system was left for another 10 minutes after the third insult. Afterward, four pieces of Coffi collagen sheets (Viscofan USA) (10 cm x 10 cm) were placed on top of the testing equipment. A thin plexiglass plate and a weight were placed on top of the Coffi sheets for 1 minute. The plexiglass plate and the weight exerted a total of 0.86 kPa on the 20 cm x 6.4 cm area. Afterward, the Coffi sheets were weighed to determine the rewet. The acquisition time and rewet results were averages of three measurements.

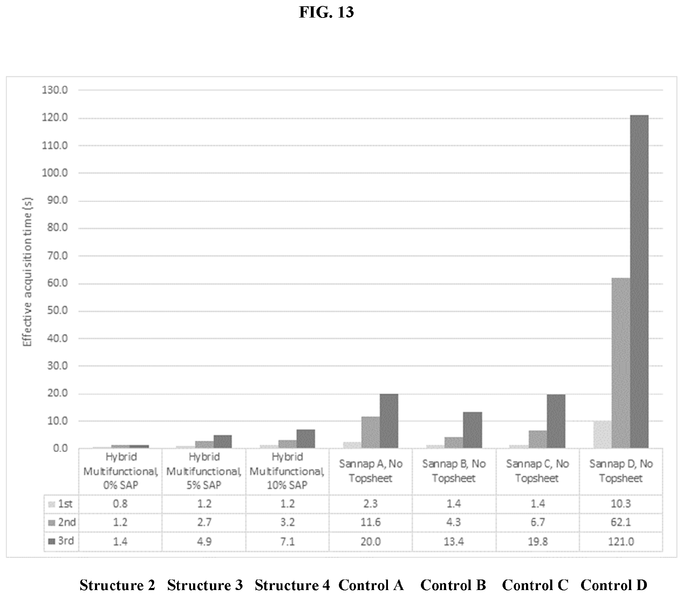

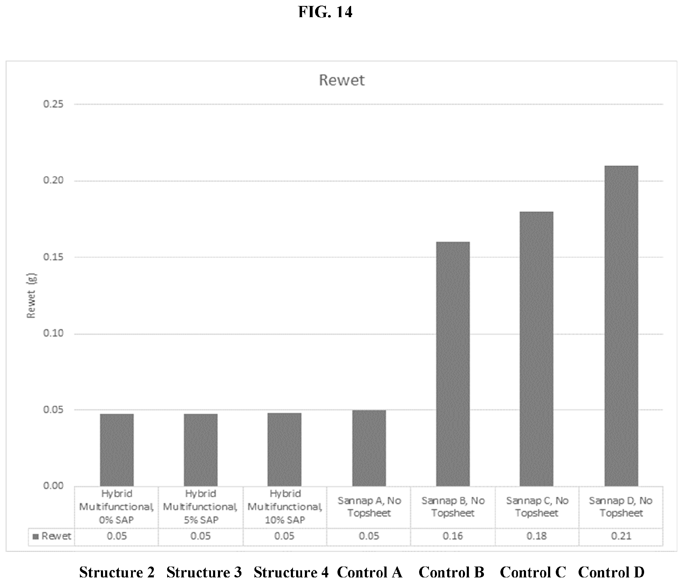

The test results are provided in FIGS. 13 and 14 and Tables 17 and 18.

FIG. 13 graphically illustrates the Effective Acquisition Times (EAT) obtained for each of the tested experimental multifunctional unitary structures (Structures 2-4) and for the control commercial sanitary napkins (Control A-D) as provided in Table 17. The EAT values were the differences between the Total Acquisition Times (TAT) and the Insult Times (IT). Each insult volume was 4 mL and the liquid were delivered at a rate of 10 mL/min. Thus, the duration of the IT was 24 seconds and the EAT = TAT - 24 seconds. As provided in FIG. 13 and Table 17, although the basis weights of Structures 2-4 are lower than the basis weights of the absorbent systems in the commercial products (Control A-D), the liquid acquisition performance of all experimental structures was higher than that of the commercial sanitary napkins including original absorbent materials.

**Table 17. Effective Acquisition Time (s)**

| | Structure 2 | Structure 3 | Structure 4 | Control A | Control B | Control C | Control D |
|---|---|---|---|---|---|---|---|
| 1 | 0.8 | 1.2 | 1.2 | 2.3 | 1.4 | 1.4 | 10.3 |
| 2 | 1.2 | 2.7 | 3.2 | 11.6 | 4.3 | 6.7 | 62.1 |
| 3 | 1.4 | 4.9 | 7.1 | 20.0 | 13.4 | 19.8 | 121.0 |

FIG. 14 graphically illustrates the rewet results obtained for each of the tested experimental multifunctional unitary structures and for the control commercial sanitary napkins as provided in Table 18. As provided in FIG. 14 and Table 18, although the basis weights of Structures 2-4 are lower than the basis weights of the absorbent systems contained in the commercial products (Control A-D) and Structures 2-4 include no or relatively low amounts of SAP, the rewet of all of Structures 2-4 provided increased performance (e.g., lower rewet values) than the commercial sanitary napkins including original absorbent materials.

**Table 18. Rewet**

| | Structure 2 | Structure 3 | Structure 4 | Control A | Control B | Control C | Control D |
|---|---|---|---|---|---|---|---|
| Rewet (g) | 0.05 | 0.05 | 0.05 | 0.05 | 0.16 | 0.18 | 0.21 |

### Wicking Distance Testing

Each structure was further tested for wicking after Total Acquisition Times #1, #2, and #3 and rewet measurements were completed. The samples were reversed, such that the underside of the core was on top. A standard metric ruler was used to measure the visible stain lengthwise along the structure. The measurement was taken from the outer edge of the stain on one side to the outer edge of the stain on the other side, parallel to the long edge of the structure.

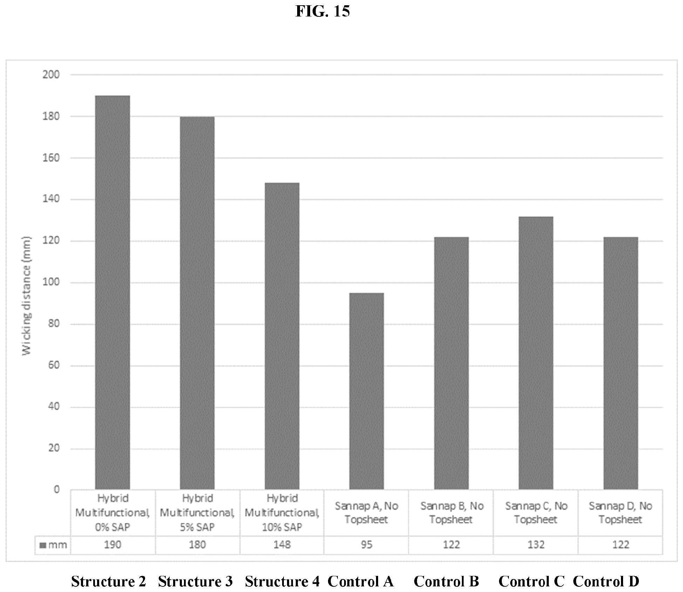

The test results are provided in FIG. 15 and Table 19.

FIG. 15 graphically illustrates the wicking results obtained for each of the tested experimental multifunctional unitary structures and for the control commercial sanitary napkins as provided in Table 19. As provided in FIG. 15 and Table 19, the wicking distances measured for all of Samples 2-4 were longer than that of the commercial sanitary napkins including original absorbent materials (Control A-D). Longer wicking distances provide for better utilization of the absorbent materials included in a personal hygiene product during its use.

**Table 19. Wicking Distance**

| | Structure 2 | Structure 3 | Structure 4 | Control A | Control B | Control C | Control D |
|---|---|---|---|---|---|---|---|
| Wicking Distance (mm) | 190 | 180 | 148 | 95 | 122 | 132 | 122 |

In addition to the various embodiments depicted and claimed, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the systems and methods of the disclosed subject matter without departing from the spirit or scope of the disclosed subject matter. Thus, it is intended that the disclosed subject matter include modifications and variations that are within the scope of the appended claims and their equivalents.

Various patents and patent applications are cited herein, the contents of which are hereby incorporated by reference herein in their entireties.
The present invention is further directed to the following embodiments:
1. A multi-layer nonwoven material comprising:
   a first layer comprising cellulose fibers and bicomponent fibers; and
   a second layer adjacent to the first layer comprising fine cellulose fibers and bicomponent fibers,
   wherein at least a portion of the first and second layers are coated with a binder.
2. The multi-layer nonwoven material of embodiment 1, wherein the nonwoven material has an effective acquisition time of about 40 seconds or less.
3. The multi-layer nonwoven material of embodiment 2, wherein the nonwoven material has an effective acquisition time of about 20 seconds or less.
4. The multi-layer nonwoven material of embodiment 1, wherein the nonwoven material has a wicking distance of at least 85 mm.
5. The multi-layer nonwoven material of embodiment 4, wherein the nonwoven material has a wicking distance of at least 140 mm.
6. The multi-layer nonwoven material of embodiment 1, wherein the nonwoven material has a rewet value of about 0.20 g or less.
7. The multi-layer nonwoven material of embodiment 6, wherein the nonwoven material has a rewet value of about 0.15 g or less.
8. The multi-layer nonwoven material of embodiment 1, wherein the nonwoven material has a retention before leak of at least about 3.0 g.
9. The multi-layer nonwoven material of embodiment 1, wherein the fine cellulose fibers include eucalyptus pulp.
10. The multi-layer nonwoven material of embodiment 1, further comprising a first intermediate layer including superabsorbent polymer (SAP) disposed between the first and second layers.
11. The multi-layer nonwoven material of embodiment 10, further comprising a second intermediate layer including cellulose fibers and bicomponent fibers disposed between the first layer and the first intermediate layer.
12. An absorbent article comprising the multi-layer nonwoven material of embodiment 1.
13. A multi-layer nonwoven material comprising:
   a first layer comprising cellulose fibers and synthetic fibers;
   a second layer adjacent to the first layer comprising cellulose fibers and synthetic fibers;
   a third layer adjacent to the second layer comprising superabsorbent polymer (SAP); and
   a fourth layer adjacent to the third layer comprising fine cellulose fibers and synthetic fibers,
   wherein at least a portion of the first and fourth layers are coated with a binder.
14. The multi-layer nonwoven material of embodiment 13, wherein the fine cellulose fibers comprise eucalyptus pulp.
15. The multi-layer nonwoven material of embodiment 13, wherein the synthetic fibers comprise bicomponent fibers.
16. An absorbent article comprising the multi-layer nonwoven material of embodiment 13.
17. A multi-layer nonwoven material comprising:
   a first layer comprising long fibers; and
   a second layer adjacent to the first layer comprising short fibers,
   wherein at least a portion of the second layer is coated with a binder.
18. The multi-layer nonwoven material of embodiment 17, wherein the nonwoven material has an effective acquisition time about 15 seconds or less.
19. The multi-layer nonwoven material of embodiment 18, wherein the nonwoven material has an effective acquisition time of about 1 second or less.
20. The multi-layer nonwoven material of embodiment 17, wherein the nonwoven material has a rewet value of about 0.5 g or less.
21. The multi-layer nonwoven material of embodiment 20, wherein the nonwoven material has a rewet value of about 0.05 g or less.
22. The multi-layer nonwoven material of embodiment 17, wherein the nonwoven material has a wicking distance of at least about 140 mm.
23. The multi-layer nonwoven material of embodiment 22, wherein the nonwoven material has a wicking distance of at least about 180 mm.
24. The multi-layer nonwoven material of embodiment 17, wherein the long fibers comprise synthetic fibers, regenerated cellulose fibers, or combinations thereof.
25. The multi-layer nonwoven material of embodiment 24, wherein the long fibers comprise synthetic fibers formed as a carded web.
26. The multi-layer nonwoven material of embodiment 17, wherein the short fibers comprise synthetic fibers, cellulose fibers, regenerated cellulose fibers, or combinations thereof.
27. The multi-layer nonwoven material of embodiment 17, wherein the long fibers have a length of between about 8 mm and about 70 mm.
28. The multi-layer nonwoven material of embodiment 17, wherein the short fibers have a length of between about 1 mm and about 8 mm.
29. An absorbent article comprising the multi-layered nonwoven material of embodiment 17.
30. A multi-layer nonwoven material comprising:
   a first layer comprising synthetic fibers formed as a carded web; and
   a second layer adjacent to the first layer comprising cellulose fibers,
   wherein at least a portion of the second layer is coated with a binder.
31. The multi-layer nonwoven material of embodiment 30, wherein the synthetic fibers have a length of between about 8 mm and about 70 mm.
32. An absorbent article comprising the multi-layer nonwoven material of embodiment 30.
33. A multi-layer nonwoven material comprising:
   a first layer comprising synthetic fibers formed as a carded web;
   a second layer adjacent to the first layer comprising cellulose fibers and bicomponent fibers; and
   a third layer adjacent to the second layer comprising cellulose fibers and bicomponent fibers,
   wherein at least a portion of the third layer is coated with a binder.
34. The multi-layer nonwoven material of embodiment 33, wherein the synthetic fibers have a length of between about 8 mm and about 70 mm.
35. The multi-layer nonwoven material of embodiment 33, wherein the cellulose fibers of the third layer comprise fine cellulose fibers.
36. The multi-layer nonwoven material of embodiment 35, wherein the cellulose fibers of the third layer comprise eucalyptus pulp.
37. An absorbent article comprising the multi-layer nonwoven material of embodiment 33.
38. A multi-layer nonwoven material comprising:
   a first layer comprising synthetic fibers formed as a carded web;
   a second layer adjacent to the first layer comprising cellulose fibers and bicomponent fibers;
   a third layer adjacent to the second layer comprising superabsorbent polymer (SAP); and
   a fourth adjacent to the third layer comprising cellulose fibers, bicomponent fibers,
   wherein at least a portion of the fourth layer is coated with a binder.
39. The multi-layer nonwoven material of embodiment 38, wherein the synthetic fibers have a length of between about 8 mm and about 70 mm.
40. The multi-layer nonwoven material of embodiment 38, wherein the cellulose fibers of the fourth layer comprise fine cellulose fibers.
41. The multi-layer nonwoven material of embodiment 40, wherein the cellulose fibers of the fourth layer comprise eucalyptus pulp.
42. An absorbent article comprising the multi-layer nonwoven material of embodiment 38.
43. A multi-layer nonwoven material comprising:
   a first layer comprising synthetic fibers formed as a carded web; and
   a second layer comprising fine hardwood cellulose fibers, wherein the fine hardwood cellulose fibers comprise a pulp fiber coarseness ranging from 4.2 mg/100 m to 14.08 mg/100 m, and
   wherein at least a portion of the second layer is coated with a binder.
44. The multi-layer nonwoven material of embodiment 43, wherein the synthetic fibers have a length of between about 8 mm and about 70 mm.
45. The multi-layer nonwoven material of embodiment 43, wherein the second layer further comprises synthetic fibers.
46. The multi-layer nonwoven material of embodiment 45, wherein the second layer has about 5 to about 70 gsm of the fine hardwood cellulose fibers and about 15 to about 40 gsm of the synthetic fibers
47. The multi-layer nonwoven material of embodiment 43, wherein the first layer comprises about 20 to about 60 gsm of the synthetic fibers formed as the carded web.
48. An absorbent article comprising the multi-layer nonwoven material of any one of embodiments 43 to 47.
49. The multi-layer nonwoven material of embodiment 43, wherein:
   the multi-layer nonwoven material further comprises a third layer adjacent to the first layer, the third layer comprising cellulose fibers and synthetic fibers; and
   the second layer further comprises bicomponent fibers.
50. The multi-layer nonwoven material of embodiment 49, wherein the synthetic fibers of the third layer have a length of between about 8 mm and about 70 mm.
51. The multi-layer nonwoven material of embodiment 49, wherein the synthetic fibers of the third layer are bicomponent fibers.
52. The multi-layer nonwoven material of embodiment 49, wherein the cellulose fibers of the third layer comprise fine cellulose fibers.
53. The multi-layer nonwoven material of embodiment 49, wherein the fine hardwood cellulose fibers of the second layer comprise eucalyptus pulp.
54. The multi-layer nonwoven material of embodiment 49, wherein the third layer has about 5 to about 70 gsm of the cellulose fibers and about 15 to about 40 gsm of the synthetic fibers.
55. An absorbent article comprising the multi-layer nonwoven material of any one of embodiments 49 to 54.

## Claims

1. A multi-layer nonwoven material comprising: a first layer comprising long fibers; and a second layer adjacent to the first layer comprising short fibers, wherein at least a portion an outer surface of the multi-layer nonwoven material is coated with a binder.

2. The multi-layer nonwoven material of claim 1, wherein the nonwoven material has an effective acquisition time about 15 seconds or less, such as about 1 second or less.

3. The multi-layer nonwoven material of claim 1, wherein the nonwoven material has a rewet value of about 0.5 g or less, such as about 0.05 g or less.

4. The multi-layer nonwoven material of claim 1, wherein the nonwoven material has a wicking distance of at least about 140 mm, such as at least about 180 mm.

5. The multi-layer nonwoven material of claim 1, wherein the long fibers comprise synthetic fibers, regenerated cellulose fibers, or combinations thereof.

6. The multi-layer nonwoven material of claim 5, wherein the long fibers comprise synthetic fibers formed as a carded web.

7. The multi-layer nonwoven material of claim 1, wherein the short fibers comprise synthetic fibers, cellulose fibers, regenerated cellulose fibers, or combinations thereof.

8. The multi-layer nonwoven material of claim 1, wherein the long fibers have a length of between about 8 mm and about 70 mm.

9. The multi-layer nonwoven material of claim 1, wherein the short fibers have a length of between about 1 mm and about 8 mm.

10. An absorbent article comprising the multi-layered nonwoven material of claim 1.

11. The multi-layer nonwoven material of claim 1, the first layer comprises synthetic fibers formed as a carded web; the second layer adjacent to the first layer comprises cellulose fibers and bicomponent fibers; and wherein the multi-layer nonwoven material further comprises a third layer adjacent to the second layer comprising superabsorbent polymer (SAP); and a fourth adjacent to the third layer comprising cellulose fibers, bicomponent fibers, wherein at least a portion of the fourth layer is coated with a binder.

12. The multi-layer nonwoven material of claim 11, wherein the synthetic fibers have a length of between about 8 mm and about 70 mm.

13. The multi-layer nonwoven material of claim 11, wherein the cellulose fibers of the fourth layer comprise fine cellulose fibers.

14. The multi-layer nonwoven material of claim 13, wherein the cellulose fibers of the fourth layer comprise eucalyptus pulp.

15. An absorbent article comprising the multi-layer nonwoven material of claim 1.
